# EUROPEAN PATENT SPECIFICATION

(11) **EP 1 311 690 B1**
(45) Date of publication and mention of the grant of the patent: **26.07.2006**
(21) Application number: 01968061.0
(22) Date of filing: 22.08.2001
(51) Int. Cl.: C12N 15/55, C12N 9/64

(54) **HUMAN PROTEASES AND POLYNUCLEOTIDES ENCODING THE SAME**
MENSCHLICHE PROTEASEN UND FÜR DIESE KODIERENDE POLYNUKLEOTIDE
PROTEASES HUMAINES ET POLYNUCLEOTIDES CODANT LESDITES PROTEASES

(30) Priority: 22.08.2000 US 227104 P; 19.09.2000 US 233796 P
(43) Date of publication of application: 21.05.2003
(73) Proprietor: Lexicon Genetics Incorporated, The Woodlands, TX 77381-1160 (US)
(72) Inventor: WALKE, Wade, D., Spring, TX 77389 (US); HILBUN, Erin, Spring, TX 77379 (US); SCOVILLE, John, Houston, TX 77024 (US); FRIDDLE, Carl, Johan, The Woodlands, TX 77382 (US); HU, Yi, The Woodlands, TX 77381 (US); TURNER, Alexander, C., Jr., The Woodlands, TX 77381 (US)
(74) Representative: Silveston, Judith
(86) International application number: PCT/US2001/026148
(87) International publication number: WO 2002/016564

(56) References cited:
- WO-A-01/59133
- WO-A-01/83782
- WO-A-01/98468
- DATABASE EMBL [Online] SMITH D.R. ET AL.: "Homo sapiens chromosome 10 clone RP11-91A1, WORKING DRAFT SEQUENCE, 25 unordered pieces." retrieved from EBI Database accession no. AC016043 XP002192569
- DATABASE SWALL [Online] 30 May 2000 (2000-05-30) CASAS C. ET AL.: " ADAMTS-1 PRECURSOR (EC 3.4.24.-) (A DISINTEGRIN AND METALLOPROTEINASE WITH THROMBOSPONDIN MOTIFS 1) (ADAM-TS 1) (ADAM-TS1) (METH-1)." retrieved from EBI Database accession no. Q9UHI8 XP002192570
- BOR LUEN TANG ET AL: "ADAMTS: A novel family of proteases with an ADAM protease domain and thrombospondin 1 repeats" FEBS LETTERS, ELSEVIER SCIENCE PUBLISHERS, AMSTERDAM, NL, vol. 445, 26 February 1999 (1999-02-26), pages 223-225, XP002141413 ISSN: 0014-5793
- BOLZ H ET AL: "Characterization of ADAMTS14, a novel member of the ADAMTS metalloproteinase family" BIOCHIMICA ET BIOPHYSICA ACTA. GENE STRUCTURE AND EXPRESSION, ELSEVIER, AMSTERDAM, NL, vol. 1522, no. 3, 30 December 2001 (2001-12-30), pages 221-225, XP004331262 ISSN: 0167-4781
- COLIGE A. ET AL: 'Cloning and characterization of ADAMTS-14, a novel ADAMTS displaying high homology with ADAMTS-2 and ADAMTS-3' JOURNAL OF BIOLOGICAL CHEMISTRY vol. 277, no. 8, 22 February 2002, BALTIMORE, pages 5756 - 5766

### SEQUENCE LISTING

<110> LEXICON GENETICS INCORPORATED
<120> Novel Human Proteases and Polynucleotides Encoding the Same
<130> LEX-0221-PCT
<150> US 60/227,104
   <151> 2000-08-22
<150> US 60/233,796
   <151> 2000-09-19
<160> 26
<170> FastSEQ for Windows Version 4.0
<210> 1
   <211> 1356
   <212> DNA
   <213> homo sapiens
<400> 1
   atggctccac tccgcgcgct gctgtcctac ctgctgcctt tgcactgtgc gctctgcgcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg   1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccgtag                              1356
<210> 2
   <211> 451
   <212> PRT
   <213> homo sapiens
<400> 2
   Met Ala Pro Leu Arg Ala Leu Leu Ser Tyr Leu Leu Pro Leu His Cys
   1               5                   10                  15
   Ala Leu Cys Ala Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro
   450
<210> 3
   <211> 894
   <212> DNA
   <213> homo sapiens
<400> 3
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtgtgcttac agggaagtcc ataa           894
<210> 4
   <211> 297
   <212> PRT
   <213> homo sapiens
<400> 4
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Cys Leu Gln Gly Ser Pro
       290                 295
<210> 5
   <211> 1461
   <212> DNA
   <213> homo sapiens
<400> 5
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg    1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg ccacctgccc gggcggccgc tcgagcccta tagtgagtcg tattaggatg    1440
   gccgccactc cctttactta a                                               1461
<210> 6
   <211> 486
   <212> PRT
   <213> homo sapiens
<400> 6
   Met Ala Pro Leu Arg Ala Leu Leu Ser Tyr Leu Leu Pro Leu His Cys
   1               5                   10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Thr Cys Pro Gly Gly Arg Ser Ser Pro Ile Val Ser Arg Ile Arg Met
   465                 470                 475                 480
   Ala Ala Thr Pro Phe Thr
<210> 7
   <211> 3669
   <212> DNA
   <213> homo sapiens
<400> 7
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg    1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc    2460
   agcctggcct acaagtacgt catccatgag gacctgctgc cccttatcgg gagcaacaat    2520
   gtgctcctgg aggagatgga cacctatgag tgggcgctca agagctgggc cccctgcagc    2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaatg    3180
   tgtgcagcgg agccctgcac gggagacagg tctgtcttct gccagatgga agtgctcgat    3240
   cgctactgct ccattcccgg ctaccaccgg ctctgctgtg tgtcctgcat caagaaggcc    3300
   tcgggcccca accctggccc agaccctggc ccaacctcac tgcccccctt ctccactcct    3360
   ggaagcccct taccaggacc ccaggaccct gcagatgctg cagagcctcc tggaaagcca    3420
   acgggatcag aggaccatca gcatggccga gccacacagc tcccaggagc tctggataca    3480
   agctccccag ggacccagca tccctttgcc cctgagacac caatccctgg agcatcctgg    3540
   agcatctccc ctaccacccc cggggggctg ccttggggct ggactcagac acctacgcca    3600
   gtccctgagg acaaagggca acctggagaa gacctgagac ctgcccgggc ggccgctcga    3660
   gccctatag                                                            3669
<210> 8
   <211> 1222
   <212> PRT
   <213> homo sapiens
<400> 8
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
           595                 600                 605
   Thr Tyr Glu Asp Phe Arg Ala Gln Gln Cys Ala Lys Arg Asn Ser Tyr
       610                 615                 620
   Tyr Val His Gln Asn Ala Lys His Ser Trp Val Pro Tyr Glu Pro Asp
   625                 630                 635                 640
   Asp Asp Ala Gln Lys Cys Glu Leu Ile Cys Gln Ser Ala Asp Thr Gly
                   645                 650                 655
   Asp Val Val Phe Met Asn Gln Val Val His Asp Gly Thr Arg Cys Ser
               660                 665                 670
   Tyr Arg Asp Pro Tyr Ser Val Cys Ala Arg Gly Glu Cys Val Pro Val
           675                 680                 685
   Gly Cys Asp Lys Glu Val Gly Ser Met Lys Ala Asp Asp Lys Cys Gly
       690                 695                 700
   Val Cys Gly Gly Asp Asn Ser His Cys Arg Thr Val Lys Gly Thr Leu
   705                 710                 715                 720
   Gly Lys Ala Ser Lys Gln Ala Gly Ala Leu Lys Leu Val Gln Ile Pro
                   725                 730                 735
   Ala Gly Ala Arg His Ile Gln Ile Glu Ala Leu Glu Lys Ser Pro His
               740                 745                 750
   Arg Ser Val Val Lys Asn Gln Val Thr Gly Ser Phe Ile Leu Asn Pro
           755                 760                 765
   Lys Gly Lys Glu Ala Thr Ser Arg Thr Phe Thr Ala Met Gly Leu Glu
       770                 775                 780
   Trp Glu Asp Ala Val Glu Asp Ala Lys Glu Ser Leu Lys Thr Ser Gly
   785                 790                 795                 800
   Pro Leu Pro Glu Ala Ile Ala Ile Leu Ala Leu Pro Pro Thr Glu Gly
                   805                 810                 815
   Gly Pro Arg Ser Ser Leu Ala Tyr Lys Tyr Val Ile His Glu Asp Leu
               820                 825                 830
   Leu Pro Leu Ile Gly Ser Asn Asn Val Leu Leu Glu Glu Met Asp Thr
           835                 840                 845
   Tyr Glu Trp Ala Leu Lys Ser Trp Ala Pro Cys Ser Lys Ala Cys Gly
       850                 855                 860
   Gly Gly Ile Gln Phe Thr Lys Tyr Gly Cys Arg Arg Arg Arg Asp His
   865                 870                 875                 880
   His Met Val Gln Arg His Leu Cys Asp His Lys Lys Arg Pro Lys Pro
                   885                 890                 895
   Ile Arg Arg Arg Cys Asn Gln His Pro Cys Ser Gln Pro Val Trp Val
               900                 905                 910
   Thr Glu Glu Trp Gly Ala Cys Ser Arg Ser Cys Gly Lys Leu Gly Val
           915                 920                 925
   Gln Thr Arg Gly Ile Gln Cys Leu Leu Pro Leu Ser Asn Gly Thr His
       930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
               980                 985                 990
   Val Val Cys Arg Thr Asn Ala Asn Ser Leu Gly His Cys Glu Gly Asp
           995                 1000                1005
   Arg Pro Asp Thr Val Gln Val Cys Ser Leu Pro Ala Cys Gly Gly Asn
       1010                1015                1020
   His Gln Asn Ser Thr Val Arg Ala Asp Val Trp Glu Leu Gly Thr Pro
   1025                1030                1035                1040
   Glu Gly Gln Trp Val Pro Gln Ser Gly Pro Leu His Pro Ile Asn Lys
                   1045                1050                1055
   Ile Ser Ser Met Cys Ala Ala Glu Pro Cys Thr Gly Asp Arg Ser Val
               1060                1065                1070
   Phe Cys Gln Met Glu Val Leu Asp Arg Tyr Cys Ser Ile Pro Gly Tyr
           1075                1080                1085
   His Arg Leu Cys Cys Val Ser Cys Ile Lys Lys Ala Ser Gly Pro Asn
       1090                1095                1100
   Pro Gly Pro Asp Pro Gly Pro Thr Ser Leu Pro Pro Phe Ser Thr Pro
   1105                1110                1115                1120
   Gly Ser Pro Leu Pro Gly Pro Gln Asp Pro Ala Asp Ala Ala Glu Pro
                   1125                1130                1135
   Pro Gly Lys Pro Thr Gly Ser Glu Asp His Gln His Gly Arg Ala Thr
               1140                1145                1150
   Gln Leu Pro Gly Ala Leu Asp Thr Ser Ser Pro Gly Thr Gln His Pro
           1155                1160                1165
   Phe Ala Pro Glu Thr Pro Ile Pro Gly Ala Ser Trp Ser Ile Ser Pro
       1170                    1175                    1180
   Thr Thr Pro Gly Gly Leu Pro Trp Gly Trp Thr Gln Thr Pro Thr Pro
   1185                    1190                    1195                1200
   Val Pro Glu Asp Lys Gly Gln Pro Gly Glu Asp Leu Arg Pro Ala Arg
                   1205                    1210                    1215
   Ala Ala Ala Arg Ala Leu
               1220
<210> 9
   <211> 3660
   <212> DNA
   <213> homo sapiens
<400> 9
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccswywctg gcatgtgtca ccccctgagg   1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc    2460
   agcctggcct acaagtacgt catccatgag gacctgctgc cccttatcgg gagcaacaat    2520
   gtgctcctgg aggagatgga cacctatgag tgggcgctca agagctgggc cccctgcagc    2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaacg    3180
   gagccctgca cgggagacag gtctgtcttc tgccagatgg aagtgctcga tcgctactgc    3240
   tccattcccg gctaccaccg gctctgctgt gtgtcctgca tcaagaaggc ctcgggcccc    3300
   aaccctggcc cagaccctgg cccaacctca ctgcccccct tctccactcc tggaagcccc    3360
   ttaccaggac cccaggaccc tgcagatgct gcagagcctc ctggaaagcc aacgggatca    3420
   gaggaccatc agcatggccg agccacacag ctcccaggag ctctggatac aagctcccca    3480
   gggacccagc atccctttgc ccctgagaca ccaatccctg gagcatcctg gagcatctcc    3540
   cctaccaccc ccggggggct gccttggggc tggactcaga cacctacgcc agtccctgag    3600
   gacaaagggc aacctggaga agacctgaga cctgcccggg cggccgctcg agccctatag    3660
<210> 10
   <211> 1219
   <212> PRT
   <213> homo sapiens
<400> 10
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
           595                 600                 605
   Thr Tyr Glu Asp Phe Arg Ala Gln Gln Cys Ala Lys Arg Asn Ser Tyr
       610                 615                 620
   Tyr Val His Gln Asn Ala Lys His Ser Trp Val Pro Tyr Glu Pro Asp
   625                 630                 635                 640
   Asp Asp Ala Gln Lys Cys Glu Leu Ile Cys Gln Ser Ala Asp Thr Gly
                   645                 650                 655
   Asp Val Val Phe Met Asn Gln Val Val His Asp Gly Thr Arg Cys Ser
               660                 665                 670
   Tyr Arg Asp Pro Tyr Ser Val Cys Ala Arg Gly Glu Cys Val Pro Val
           675                 680                 685
   Gly Cys Asp Lys Glu Val Gly Ser Met Lys Ala Asp Asp Lys Cys Gly
       690                 695                 700
   Val Cys Gly Gly Asp Asn Ser His Cys Arg Thr Val Lys Gly Thr Leu
   705                 710                 715                 720
   Gly Lys Ala Ser Lys Gln Ala Gly Ala Leu Lys Leu Val Gln Ile Pro
                   725                 730                 735
   Ala Gly Ala Arg His Ile Gln Ile Glu Ala Leu Glu Lys Ser Pro His
               740                 745                 750
   Arg Ser Val Val Lys Asn Gln Val Thr Gly Ser Phe Ile Leu Asn Pro
           755                 760                 765
   Lys Gly Lys Glu Ala Thr Ser Arg Thr Phe Thr Ala Met Gly Leu Glu
       770                 775                 780
   Trp Glu Asp Ala Val Glu Asp Ala Lys Glu Ser Leu Lys Thr Ser Gly
   785                 790                 795                 800
   Pro Leu Pro Glu Ala Ile Ala Ile Leu Ala Leu Pro Pro Thr Glu Gly
                   805                 810                 815
   Gly Pro Arg Ser Ser Leu Ala Tyr Lys Tyr Val Ile His Glu Asp Leu
               820                 825                 830
   Leu Pro Leu Ile Gly Ser Asn Asn Val Leu Leu Glu Glu Met Asp Thr
           835                 840                 845
   Tyr Glu Trp Ala Leu Lys Ser Trp Ala Pro Cys Ser Lys Ala Cys Gly
       850                 855                 860
   Gly Gly Ile Gln Phe Thr Lys Tyr Gly Cys Arg Arg Arg Arg Asp His
   865                 870                 875                 880
   His Met Val Gln Arg His Leu Cys Asp His Lys Lys Arg Pro Lys Pro
                   885                 890                 895
   Ile Arg Arg Arg Cys Asn Gln His Pro Cys Ser Gln Pro Val Trp Val
               900                 905                 910
   Thr Glu Glu Trp Gly Ala Cys Ser Arg Ser Cys Gly Lys Leu Gly Val
           915                 920                 925
   Gln Thr Arg Gly Ile Gln Cys Leu Leu Pro Leu Ser Asn Gly Thr His
       930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
               980                 985                 990
   Val Val Cys Arg Thr Asn Ala Asn Ser Leu Gly His Cys Glu Gly Asp
           995                 1000                1005
   Arg Pro Asp Thr Val Gln Val Cys Ser Leu Pro Ala Cys Gly Gly Asn
       1010                1015                1020
   His Gln Asn Ser Thr Val Arg Ala Asp Val Trp Glu Leu Gly Thr Pro
   1025                1030                1035                1040
   Glu Gly Gln Trp Val Pro Gln Ser Gly Pro Leu His Pro Ile Asn Lys
                   1045                1050                1055
   Ile Ser Ser Thr Glu Pro Cys Thr Gly Asp Arg Ser Val Phe Cys Gln
               1060                1065                1070
   Met Glu Val Leu Asp Arg Tyr Cys Ser Ile Pro Gly Tyr His Arg Leu
           1075                1080                1085
   Cys Cys Val Ser Cys Ile Lys Lys Ala Ser Gly Pro Asn Pro Gly Pro
       1090                1095                1100
   Asp Pro Gly Pro Thr Ser Leu Pro Pro Phe Ser Thr Pro Gly Ser Pro
   1105                1110                1115                1120
   Leu Pro Gly Pro Gln Asp Pro Ala Asp Ala Ala Glu Pro Pro Gly Lys
                   1125                1130                1135
   Pro Thr Gly Ser Glu Asp His Gln His Gly Arg Ala Thr Gln Leu Pro
               1140                1145                1150
   Gly Ala Leu Asp Thr Ser Ser Pro Gly Thr Gln His Pro Phe Ala Pro
           1155                1160                1165
   Glu Thr Pro Ile Pro Gly Ala Ser Trp Ser Ile Ser Pro Thr Thr Pro
       1170                1175                1180
   Gly Gly Leu Pro Trp Gly Trp Thr Gln Thr Pro Thr Pro Val Pro Glu
   1185                1190                1195                1200
   Asp Lys Gly Gln Pro Gly Glu Asp Leu Arg Pro Ala Arg Ala Ala Ala
                   1205                1210                1215
   Arg Ala Leu
<210> 11
   <211> 3651
   <212> DNA
   <213> homo sapiens
<400> 11
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg    1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc    2460
   agcctggcct acaagtacgt catccatgag gacctgctgc cccttatcgg gagcaacaat    2520
   gtgctcctgg aggagatgga cacctatgag tgggcgctca agagctgggc cccctgcagc    2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaatg    3180
   tgtgcagcgg agccctgcac gggagacagg tctgtcttct gccagatgga agtgctcgat    3240
   cgctactgct ccattcccgg ctaccaccgg ctctgctgtg tgtcctgcat caagaaggcc    3300
   tcgggcccca accctggccc agaccctggc ccaacctcac tgcccccctt ctccactcct    3360
   ggaagcccct taccaggacc ccaggaccct gcagatgctg cagagcctcc tggaaagcca    3420
   acgggatcag aggaccatca gcatggccga gccacacagc tcccaggagc tctggataca    3480
   agctccccag ggacccagca tccctttgcc cctgagacac caatccctgg agcatcctgg    3540
   agcatctccc ctaccacccc cggggggctg ccttggggct ggactcagac acctacgcca     3600
   gtccctgacc tgcccgggcg gccgctcgag ccctatagtg agtcgtatta g             3651
<210> 12
   <211> 1216
   <212> PRT
   <213> homo sapiens
<400> 12
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
           595                 600                 605
   Thr Tyr Glu Asp Phe Arg Ala Gln Gln Cys Ala Lys Arg Asn Ser Tyr
       610                 615                 620
   Tyr Val His Gln Asn Ala Lys His Ser Trp Val Pro Tyr Glu Pro Asp
   625                 630                 635                 640
   Asp Asp Ala Gln Lys Cys Glu Leu Ile Cys Gln Ser Ala Asp Thr Gly
                   645                 650                 655
   Asp Val Val Phe Met Asn Gln Val Val His Asp Gly Thr Arg Cys Ser
               660                 665                 670
   Tyr Arg Asp Pro Tyr Ser Val Cys Ala Arg Gly Glu Cys Val Pro Val
           675                 680                 685
   Gly Cys Asp Lys Glu Val Gly Ser Met Lys Ala Asp Asp Lys Cys Gly
       690                 695                 700
   Val Cys Gly Gly Asp Asn Ser His Cys Arg Thr Val Lys Gly Thr Leu
   705                 710                 715                 720
   Gly Lys Ala Ser Lys Gln Ala Gly Ala Leu Lys Leu Val Gln Ile Pro
                   725                 730                 735
   Ala Gly Ala Arg His Ile Gln Ile Glu Ala Leu Glu Lys Ser Pro His
               740                 745                 750
   Arg Ser Val Val Lys Asn Gln Val Thr Gly Ser Phe Ile Leu Asn Pro
           755                 760                 765
   Lys Gly Lys Glu Ala Thr Ser Arg Thr Phe Thr Ala Met Gly Leu Glu
       770                 775                 780
   Trp Glu Asp Ala Val Glu Asp Ala Lys Glu Ser Leu Lys Thr Ser Gly
   785                 790                 795                 800
   Pro Leu Pro Glu Ala Ile Ala Ile Leu Ala Leu Pro Pro Thr Glu Gly
                   805                 810                 815
   Gly Pro Arg Ser Ser Leu Ala Tyr Lys Tyr Val Ile His Glu Asp Leu
               820                 825                 830
   Leu Pro Leu Ile Gly Ser Asn Asn Val Leu Leu Glu Glu Met Asp Thr
           835                 840                 845
   Tyr Glu Trp Ala Leu Lys Ser Trp Ala Pro Cys Ser Lys Ala Cys Gly
       850                 855                 860
   Gly Gly Ile Gln Phe Thr Lys Tyr Gly Cys Arg Arg Arg Arg Asp His
   865                 870                 875                 880
   His Met Val Gln Arg His Leu Cys Asp His Lys Lys Arg Pro Lys Pro
                   885                 890                 895
   Ile Arg Arg Arg Cys Asn Gln His Pro Cys Ser Gln Pro Val Trp Val
               900                 905                 910
   Thr Glu Glu Trp Gly Ala Cys Ser Arg Ser Cys Gly Lys Leu Gly Val
           915                 920                 925
   Gln Thr Arg Gly Ile Gln Cys Leu Leu Pro Leu Ser Asn Gly Thr His
       930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
               980                 985                 990
   Val Val Cys Arg Thr Asn Ala Asn Ser Leu Gly His Cys Glu Gly Asp
           995                 1000                1005
   Arg Pro Asp Thr Val Gln Val Cys Ser Leu Pro Ala Cys Gly Gly Asn
       1010                1015                1020
   His Gln Asn Ser Thr Val Arg Ala Asp Val Trp Glu Leu Gly Thr Pro
   1025                1030                1035                1040
   Glu Gly Gln Trp Val Pro Gln Ser Gly Pro Leu His Pro Ile Asn Lys
                   1045                1050                1055
   Ile Ser Ser Met Cys Ala Ala Glu Pro Cys Thr Gly Asp Arg Ser Val
               1060                1065                1070
   Phe Cys Gln Met Glu Val Leu Asp Arg Tyr Cys Ser Ile Pro Gly Tyr
           1075                1080                1085
   His Arg Leu Cys Cys Val Ser Cys Ile Lys Lys Ala Ser Gly Pro Asn
           1090                1095                1100
   Pro Gly Pro Asp Pro Gly Pro Thr Ser Leu Pro Pro Phe Ser Thr Pro
   1105                1110                1115                1120
   Gly Ser Pro Leu Pro Gly Pro Gln Asp Pro Ala Asp Ala Ala Glu Pro
                   1125                1130                1135
   Pro Gly Lys Pro Thr Gly Ser Glu Asp His Gln His Gly Arg Ala Thr
               1140                1145                1150
   Gln Leu Pro Gly Ala Leu Asp Thr Ser Ser Pro Gly Thr Gln His Pro
           1155                1160                1165
   Phe Ala Pro Glu Thr Pro Ile Pro Gly Ala Ser Trp Ser Ile Ser Pro
       1170                1175                1180
   Thr Thr Pro Gly Gly Leu Pro Trp Gly Trp Thr Gln Thr Pro Thr Pro
   1185                1190                1195                1200
   Val Pro Asp Leu Pro Gly Arg Pro Leu Glu Pro Tyr Ser Glu Ser Tyr
                   1205                1210                1215
<210> 13
   <211> 3642
   <212> DNA
   <213> homo sapiens
<400> 13
   atggctccac tccgcgcgct gctgtcctac ctgctgcctt tgcactgtgc gctctgcrcc 60
   gccgcgggca gccggacccc agagctgcac ctctctggaa agctcagtga ctatggtgtg 120
   acagtgccct gcagcacaga ctttcgggga cgcttcctct cccacgtggt gtctggccca 180
   gcagcagcct ctgcagggag catggtagtg gacacgccac ccacactacc acgacactcc 240
   agtcacctcc gggtggctcg cagccctctg cacccaggag ggaccctgtg gcctggcagg 300
   gtggggcgcc actccctcta cttcaatgtc actgttttcg ggaaggaact gcacttgcgc 360
   ctgcggccca atcggaggtt ggtagtgcca ggatcctcag tggagtggca ggaggatttt 420
   cgggagctgt tccggcagcc cttacggcag gagtgtgtgt acactggagg tgtcactgga 480
   atgcctgggg cagctgttgc catcagcaac tgtgacggat tggcgggcct catccgcaca 540
   gacagcaccg acttcttcat tgagcctctg gagcggggcc agcaggagaa ggaggccagc 600
   gggaggacac atgtggtgta ccgccgggag gccgtccagc aggagtgggc agaacctgac 660
   ggggacctgc acaatgaagc ctttggcctg ggagaccttc ccaacctgct gggcctggtg 720
   ggggaccagc tgggcgacac agagcggaag cggcggcatg ccaagccagg cagctacagc 780
   atcgaggtgc tgctggtggt ggacgactcg gtggttcgct tccatggcaa ggagcatgtg 840
   cagaactatg tcctcaccct catgaatatc gtagatgaga tttaccacga tgagtccctg 900
   ggggttcata taaatattgc cctcgtccgc ttgatcatgg ttggctaccg acagtccctg 960
   agcctgatcg agcgcgggaa cccctcacgc agcctggagc aggtgtgtcg ctgggcacac 1020
   tcccagcagc gccaggaccc cagccacgct gagcaccatg accacgttgt gttcctcacc 1080
   cggcaggact ttgggccctc agggtatgca cccgtcactg gcatgtgtca ccccctgagg 1140
   agctgtgccc tcaaccatga ggatggcttc tcctcagcct tcgtgatagc tcatgagacc 1200
   ggccacgtgc tcggcatgga gcatgacggt caggggaatg gctgtgcaga tgagaccagc 1260
   ctgggcagcg tcatggcgcc cctggtgcag gctgccttcc accgcttcca ttggtcccgc 1320
   tgcagcaagc tggagctcag ccgctacctc ccctcctacg actgcctcct cgatgacccc 1380
   tttgatcctg cctggcccca gcccccagag ctgcctggga tcaactactc aatggatgag 1440
   cagtgccgct ttgactttgg cagtggctac cagacctgct tggcattcag gacctttgag 1500
   ccctgcaagc agctgtggtg cagccatcct gacaaccmgt ayttctgcaa gaccaagaag 1560
   gggcccccgc tggatgggac tgagtgtgca cccggcaagt ggtgcttcaa aggtcactgc 1620
   atctggaagt cgccggagca gacatatggc caggatggag gctggagctc ctggaccaag 1680
   tttgggtcat gttcgcggtc atgtgggggc ggggtgcgat cccgcagccg gagctgcaac 1740
   aacccctccc cagcctatgg aggccgccyg tgcttagggc ccatgttcga gtaccaggtc 1800
   tgcaacagcg aggagtgccc tgggacctac gaggacttcc gggcccagca gtgtgccaag 1860
   cgcaactcst actatgtgca ccagaatgcc aagcacagst gggtgcccta cgagcctgac 1920
   gatgacgccc agaagtgtga gctgatctgc cagtcggcgg acacrgggga cgtggtgttc 1980
   atgaaccagg tggttcacga tgggacacgc tgcagctacc gggacccata cagcgtctgt 2040
   gcgcgtggcg agtgtgtgcc tgtcggctgt gacaaggagg tggggtccat gaaggcggat 2100
   gacaagtgtg gagtctgcgg gggtgacaac tcccactgca ggactgtgaa ggggacgctg 2160
   ggcaaggcct ccaagcaggc aggagctctc aagctggtgc agatcccagc aggtgccagg 2220
   cacatccaga ttgaggcact ggagaagtcc ccccaccgsw ywgtggtgaa gaaccaggtc 2280
   accggcagct tcatcctcaa ccccaagggc aaggaagcca caagccggac cttcaccgcc 2340
   atgggcctgg agtgggagga tgcggtggag gatgccaagg aaagcctcaa gaccagcggg 2400
   cccctgcctg aagccattgc catcctggct ctccccccaa ctgagggtgg cccccgcagc 2460
   agcctggcct acaagtacgt catccatgag gacctgctgc cccttatcgg gagcaacaat 2520
   gtgctcctgg aggagatgga cacctatgag tgggcgctca agagctgggc cccctgcagc 2580
   aaggcctgtg gaggagggat ccagttcacc aaatacggct gccggcgcag acgagaccac 2640
   cacatggtgc agcgacacct gtgtgaccac aagaagaggc ccaagcccat ccgccggcgc 2700
   tgcaaccagc acccgtgctc tcagcctgtg tgggtgacgg aggagtgggg tgcctgcagc 2760
   cggagctgtg ggaagctggg ggtgcagaca cgggggatac agtgcctgct gcccctctcc 2820
   aatggaaccc acaaggtcat gccggccaaa gcctgcgccg gggaccggcc tgaggcccga 2880
   cggccctgtc tccgagtgcc ctgcccagcc cagtggaggc tgggagcctg gtcccagtgc 2940
   tctgccacct gtggagaggg catccagcag cggcaggtgg tgtgcaggac caacgccaac 3000
   agcctcgggc attgcgaggg ggataggcca gacactgtcc aggtctgcar cctgcccgcc 3060
   tgtggaggaa atcaccagaa ctccacggtg agggccgatg tctgggaact tgggacgcca 3120
   gaggggcagt gggtgccaca atctgraccc ctacatccca ttaacaagat atcatcaacg 3180
   gagccctgca cgggagacag gtctgtcttc tgccagatgg aagtgctcga tcgctactgc 3240
   tccattcccg gctaccaccg gctctgctgt gtgtcctgca tcaagaaggc ctcgggcccc 3300
   aaccctggcc cagaccctgg cccaacctca ctgcccccct tctccactcc tggaagcccc 3360
   ttaccaggac cccaggaccc tgcagatgct gcagagcctc ctggaaagcc aacgggatca 3420
   gaggaccatc agcatggccg agccacacag ctcccaggag ctctggatac aagctcccca 3480
   gggacccagc atccctttgc ccctgagaca ccaatccctg gagcatcctg gagcatctcc 3540
   cctaccaccc ccggggggct gccttggggc tggactcaga cacctacgcc agtccctgac 3600
   ctgcccgggc ggccgctcga gccctatagt gagtcgtatt ag 3642
<210> 14
   <211> 1213
   <212> PRT
   <213> homo sapiens
<400> 14
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
           595                 600                 605
   Thr Tyr Glu Asp Phe Arg Ala Gln Gln Cys Ala Lys Arg Asn Ser Tyr
       610                 615                 620
   Tyr Val His Gln Asn Ala Lys His Ser Trp Val Pro Tyr Glu Pro Asp
   625                 630                 635                 640
   Asp Asp Ala Gln Lys Cys Glu Leu Ile Cys Gln Ser Ala Asp Thr Gly
                   645                 650                 655
   Asp Val Val Phe Met Asn Gln Val Val His Asp Gly Thr Arg Cys Ser
               660                 665                 670
   Tyr Arg Asp Pro Tyr Ser Val Cys Ala Arg Gly Glu Cys Val Pro Val
           675                 680                 685
   Gly Cys Asp Lys Glu Val Gly Ser Met Lys Ala Asp Asp Lys Cys Gly
       690                 695                 700
   Val Cys Gly Gly Asp Asn Ser His Cys Arg Thr Val Lys Gly Thr Leu
   705                 710                 715                 720
   Gly Lys Ala Ser Lys Gln Ala Gly Ala Leu Lys Leu Val Gln Ile Pro
                   725                 730                 735
   Ala Gly Ala Arg His Ile Gln Ile Glu Ala Leu Glu Lys Ser Pro His
               740                 745                 750
   Arg Ser Val Val Lys Asn Gln Val Thr Gly Ser Phe Ile Leu Asn Pro
           755                 760                 765
   Lys Gly Lys Glu Ala Thr Ser Arg Thr Phe Thr Ala Met Gly Leu Glu
       770                 775                 780
   Trp Glu Asp Ala Val Glu Asp Ala Lys Glu Ser Leu Lys Thr Ser Gly
   785                 790                 795                 800
   Pro Leu Pro Glu Ala Ile Ala Ile Leu Ala Leu Pro Pro Thr Glu Gly
                   805                 810                 815
   Gly Pro Arg Ser Ser Leu Ala Tyr Lys Tyr Val Ile His Glu Asp Leu
               820                 825                 830
   Leu Pro Leu Ile Gly Ser Asn Asn Val Leu Leu Glu Glu Met Asp Thr
           835                 840                 845
   Tyr Glu Trp Ala Leu Lys Ser Trp Ala Pro Cys Ser Lys Ala Cys Gly
       850                 855                 860
   Gly Gly Ile Gln Phe Thr Lys Tyr Gly Cys Arg Arg Arg Arg Asp His
   865                 870                 875                 880
   His Met Val Gln Arg His Leu Cys Asp His Lys Lys Arg Pro Lys Pro
                   885                 890                 895
   Ile Arg Arg Arg Cys Asn Gln His Pro Cys Ser Gln Pro Val Trp Val
               900                 905                 910
   Thr Glu Glu Trp Gly Ala Cys Ser Arg Ser Cys Gly Lys Leu Gly Val
           915                 920                 925
   Gln Thr Arg Gly Ile Gln Cys Leu Leu Pro Leu Ser Asn Gly Thr His
       930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
               980                 985                 990
   Val Val Cys Arg Thr Asn Ala Asn Ser Leu Gly His Cys Glu Gly Asp
           995                 1000                1005
   Arg Pro Asp Thr Val Gln Val Cys Ser Leu Pro Ala Cys Gly Gly Asn
       1010                1015                1020
   His Gln Asn Ser Thr Val Arg Ala Asp Val Trp Glu Leu Gly Thr Pro
   1025                1030                1035                1040
   Glu Gly Gln Trp Val Pro Gln Ser Gly Pro Leu His Pro Ile Asn Lys
                   1045                1050                1055
   Ile Ser Ser Thr Glu Pro Cys Thr Gly Asp Arg Ser Val Phe Cys Gln
               1060                1065                1070
   Met Glu Val Leu Asp Arg Tyr Cys Ser Ile Pro Gly Tyr His Arg Leu
   1075 1080 1085
   Cys Cys Val Ser Cys Ile Lys Lys Ala Ser Gly Pro Asn Pro Gly Pro
   1090 1095 1100
   Asp Pro Gly Pro Thr Ser Leu Pro Pro Phe Ser Thr Pro Gly Ser Pro
   1105 1110 1115 1120
   Leu Pro Gly Pro Gln Asp Pro Ala Asp Ala Ala Glu Pro Pro Gly Lys
   1125 1130 1135
   Pro Thr Gly Ser Glu Asp His Gln His Gly Arg Ala Thr Gln Leu Pro
   1140 1145 1150
   Gly Ala Leu Asp Thr Ser Ser Pro Gly Thr Gln His Pro Phe Ala Pro
   1155 1160 1165
   Glu Thr Pro Ile Pro Gly Ala Ser Trp Ser Ile Ser Pro Thr Thr Pro
   1170 1175 1180
   Gly Gly Leu Pro Trp Gly Trp Thr Gln Thr Pro Thr Pro Val Pro Asp
   1185 1190 1195 1200
   Leu Pro Gly Arg Pro Leu Glu Pro Tyr Ser Glu Ser Tyr
   1205 1210
<210> 15
   <211> 3708
   <212> DNA
   <213> homo sapiens
<400> 15
   atggctccac tccgcgcgct gctgtcctac ctgctgcctt tgcactgtgc gctctgcrcc      60
   gccgcgggca gccggacccc agagctgcac ctctctggaa agctcagtga ctatggtgtg     120
   acagtgccct gcagcacaga ctttcgggga cgcttcctct cccacgtggt gtctggccca     180
   gcagcagcct ctgcagggag catggtagtg gacacgccac ccacactacc acgacactcc     240
   agtcacctcc gggtggctcg cagccctctg cacccaggag ggaccctgtg gcctggcagg     300
   gtggggcgcc actccctcta cttcaatgtc actgttttcg ggaaggaact gcacttgcgc     360
   ctgcggccca atcggaggtt ggtagtgcca ggatcctcag tggagtggca ggaggatttt     420
   cgggagctgt tccggcagcc cttacggcag gagtgtgtgt acactggagg tgtcactgga     480
   atgcctgggg cagctgttgc catcagcaac tgtgacggat tggcgggcct catccgcaca     540
   gacagcaccg acttcttcat tgagcctctg gagcggggcc agcaggagaa ggaggccagc     600
   gggaggacac atgtggtgta ccgccgggag gccgtccagc aggagtgggc agaacctgac     660
   ggggacctgc acaatgaagc ctttggcctg ggagaccttc ccaacctgct gggcctggtg     720
   ggggaccagc tgggcgacac agagcggaag cggcggcatg ccaagccagg cagctacagc     780
   atcgaggtgc tgctggtggt ggacgactcg gtggttcgct tccatggcaa ggagcatgtg     840
   cagaactatg tcctcaccct catgaatatc gtagatgaga tttaccacga tgagtccctg     900
   ggggttcata taaatattgc cctcgtccgc ttgatcatgg ttggctaccg acagtccctg     960
   agcctgatcg agcgcgggaa cccctcacgc agcctggagc aggtgtgtcg ctgggcacac    1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg    1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc     2460
   agcctggcct acaagtacgt catccatgag gacctgctgc cccttatcgg gagcaacaat    2520
   gtgctcctgg aggagatgga cacctatgag tgggcgctca agagctgggc cccctgcagc    2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaatg    3180
   tgtgcagcgg agccctgcac gggagacagg tctgtcttct gccagatgga agtgctcgat    3240
   cgctactgct ccattcccgg ctaccaccgg ctctgctgtg tgtcctgcat caagaaggcc    3300
   tcgggcccca accctggccc agaccctggc ccaacctcac tgcccccctt ctccactcct    3360
   ggaagcccct taccaggacc ccaggaccct gcagatgctg cagagcctcc tggaaagcca    3420
   acgggatcag aggaccatca gcatggccga gccacacagc tcccaggagc tctggataca    3480
   agctccccag ggacccagca tccctttgcc cctgagacac caatccctgg agcatcctgg    3540
   agcatctccc ctaccacccc cggggggctg ccttggggct ggactcagac acctacgcca     3600
   gtccctgagg acaaagggca acctggagaa gacctgagac atcccggcac cagcctccct    3660
   gctgacctgc ccgggcggcc gcccgagccc tatagtgagt ctgattag                  3708
<210> 16
   <211> 1235
   <212> PRT
   <213> homo sapiens
<400> 16
   Met Ala Pro Leu Arg Ala Leu Leu Ser Tyr Leu Leu Pro Leu His Cys
    1              5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
   595 600 605
   Thr Tyr Glu Asp Phe Arg Ala Gln Gln Cys Ala Lys Arg Asn Ser Tyr
   610 615 620
   Tyr Val His Gln Asn Ala Lys His Ser Trp Val Pro Tyr Glu Pro Asp
   625 630 635 640
   Asp Asp Ala Gln Lys Cys Glu Leu Ile Cys Gln Ser Ala Asp Thr Gly
   645 650 655
   Asp Val Val Phe Met Asn Gln Val Val His Asp Gly Thr Arg Cys Ser
   660 665 670
   Tyr Arg Asp Pro Tyr Ser Val Cys Ala Arg Gly Glu Cys Val Pro Val
   675 680 685
   Gly Cys Asp Lys Glu Val Gly Ser Met Lys Ala Asp Asp Lys Cys Gly
   690 695 700
   Val Cys Gly Gly Asp Asn Ser His Cys Arg Thr Val Lys Gly Thr Leu
   705 710 715 720
   Gly Lys Ala Ser Lys Gln Ala Gly Ala Leu Lys Leu Val Gln Ile Pro
   725 730 735
   Ala Gly Ala Arg His Ile Gln Ile Glu Ala Leu Glu Lys Ser Pro His
   740 745 750
   Arg Ser Val Val Lys Asn Gln Val Thr Gly Ser Phe Ile Leu Asn Pro
   755 760 765
   Lys Gly Lys Glu Ala Thr Ser Arg Thr Phe Thr Ala Met Gly Leu Glu
   770 775 780
   Trp Glu Asp Ala Val Glu Asp Ala Lys Glu Ser Leu Lys Thr Ser Gly
   785 790 795 800
   Pro Leu Pro Glu Ala Ile Ala Ile Leu Ala Leu Pro Pro Thr Glu Gly
   805 810 815
   Gly Pro Arg Ser Ser Leu Ala Tyr Lys Tyr Val Ile His Glu Asp Leu
   820 825 830
   Leu Pro Leu Ile Gly Ser Asn Asn Val Leu Leu Glu Glu Met Asp Thr
   835 840 845
   Tyr Glu Trp Ala Leu Lys Ser Trp Ala Pro Cys Ser Lys Ala Cys Gly
   850 855 860
   Gly Gly Ile Gln Phe Thr Lys Tyr Gly Cys Arg Arg Arg Arg Asp His
   865 870 875 880
   His Met Val Gln Arg His Leu Cys Asp His Lys Lys Arg Pro Lys Pro
   885 890 895
   Ile Arg Arg Arg Cys Asn Gln His Pro Cys Ser Gln Pro Val Trp Val
   900 905 910
   Thr Glu Glu Trp Gly Ala Cys Ser Arg Ser Cys Gly Lys Leu Gly Val
   915 920 925
   Gln Thr Arg Gly Ile Gln Cys Leu Leu Pro Leu Ser Asn Gly Thr His
   930 935 940
   Lys Val Met Pro Ala Lys Ala Cys Ala Gly Asp Arg Pro Glu Ala Arg
   945 950 955 960
   Arg Pro Cys Leu Arg Val Pro Cys Pro Ala Gln Trp Arg Leu Gly Ala
   965 970 975
   Trp Ser Gln Cys Ser Ala Thr Cys Gly Glu Gly Ile Gln Gln Arg Gln
   980 985 990
   Val Val Cys Arg Thr Asn Ala Asn Ser Leu Gly His Cys Glu Gly Asp
   995 1000 1005
   Arg Pro Asp Thr Val Gln Val Cys Ser Leu Pro Ala Cys Gly Gly Asn
   1010 1015 1020
   His Gln Asn Ser Thr Val Arg Ala Asp Val Trp Glu Leu Gly Thr Pro
   1025 1030 1035 1040
   Glu Gly Gln Trp Val Pro Gln Ser Gly Pro Leu His Pro Ile Asn Lys
                   1045                1050                1055
   Ile Ser Ser Met Cys Ala Ala Glu Pro Cys Thr Gly Asp Arg Ser Val
               1060                1065                1070
   Phe Cys Gln Met Glu Val Leu Asp Arg Tyr Cys Ser Ile Pro Gly Tyr
           1075                1080                1085
   His Arg Leu Cys Cys Val Ser Cys Ile Lys Lys Ala Ser Gly Pro Asn
       1090                1095                1100
   Pro Gly Pro Asp Pro Gly Pro Thr Ser Leu Pro Pro Phe Ser Thr Pro
   1105                1110                1115                1120
   Gly Ser Pro Leu Pro Gly Pro Gln Asp Pro Ala Asp Ala Ala Glu Pro
                   1125                1130                1135
   Pro Gly Lys Pro Thr Gly Ser Glu Asp His Gln His Gly Arg Ala Thr
               1140                1145                1150
   Gln Leu Pro Gly Ala Leu Asp Thr Ser Ser Pro Gly Thr Gln His Pro
           1155                1160                1165
   Phe Ala Pro Glu Thr Pro Ile Pro Gly Ala Ser Trp Ser Ile Ser Pro
       1170                1175                1180
   Thr Thr Pro Gly Gly Leu Pro Trp Gly Trp Thr Gln Thr Pro Thr Pro
   1185                1190                1195                1200
   Val Pro Glu Asp Lys Gly Gln Pro Gly Glu Asp Leu Arg His Pro Gly
                   1205                1210                1215
   Thr Ser Leu Pro Ala Asp Leu Pro Gly Arg Pro Pro Glu Pro Tyr Ser
               1220                1225                1230
   Glu Ser Asp
           1235
<210> 17
   <211> 3699
   <212> DNA
   <213> homo sapiens
<400> 17
   atggctccac tccgcgcgct gctgtcctac ctgctgcctt tgcactgtgc gctctgcrcc    60
   gccgcgggca gccggacccc agagctgcac ctctctggaa agctcagtga ctatggtgtg   120
   acagtgccct gcagcacaga ctttcgggga cgcttcctct cccacgtggt gtctggccca   180
   gcagcagcct ctgcagggag catggtagtg gacacgccac ccacactacc acgacactcc   240
   agtcacctcc gggtggctcg cagccctctg cacccaggag ggaccctgtg gcctggcagg   300
   gtggggcgcc actccctcta cttcaatgtc actgttttcg ggaaggaact gcacttgcgc   360
   ctgcggccca atcggaggtt ggtagtgcca ggatcctcag tggagtggca ggaggatttt   420
   cgggagctgt tccggcagcc cttacggcag gagtgtgtgt acactggagg tgtcactgga   480
   atgcctgggg cagctgttgc catcagcaac tgtgacggat tggcgggcct catccgcaca   540
   gacagcaccg acttcttcat tgagcctctg gagcggggcc agcaggagaa ggaggccagc   600
   gggaggacac atgtggtgta ccgccgggag gccgtccagc aggagtgggc agaacctgac   660
   ggggacctgc acaatgaagc ctttggcctg ggagaccttc ccaacctgct gggcctggtg   720
   ggggaccagc tgggcgacac agagcggaag cggcggcatg ccaagccagg cagctacagc   780
   atcgaggtgc tgctggtggt ggacgactcg gtggttcgct tccatggcaa ggagcatgtg   840
   cagaactatg tcctcaccct catgaatatc gtagatgaga tttaccacga tgagtccctg   900
   ggggttcata taaatattgc cctcgtccgc ttgatcatgg ttggctaccg acagtccctg   960
   agcctgatcg agcgcgggaa cccctcacgc agcctggagc aggtgtgtcg ctgggcacac  1020
   tcccagcagc gccaggaccc cagccacgct gagcaccatg accacgttgt gttcctcacc  1080
   cggcaggact ttgggccctc agggtatgca cccgtcactg gcatgtgtca ccccctgagg  1140
   agctgtgccc tcaaccatga ggatggcttc tcctcagcct tcgtgatagc tcatgagacc  1200
   ggccacgtgc tcggcatgga gcatgacggt caggggaatg gctgtgcaga tgagaccagc  1260
   ctgggcagcg tcatggcgcc cctggtgcag gctgccttcc accgcttcca ttggtcccgc  1320
   tgcagcaagc tggagctcag ccgctacctc ccctcctacg actgcctcct cgatgacccc  1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc    2460
   agcctggcct acaagtacgt catccatgag gacctgctgc cccttatcgg gagcaacaat    2520
   gtgctcctgg aggagatgga cacctatgag tgggcgctca agagctgggc cccctgcagc     2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaacg    3180
   gagccctgca cgggagacag gtctgtcttc tgccagatgg aagtgctcga tcgctactgc    3240
   tccattcccg gctaccaccg gctctgctgt gtgtcctgca tcaagaaggc ctcgggcccc    3300
   aaccctggcc cagaccctgg cccaacctca ctgcccccct tctccactcc tggaagcccc   3360
   ttaccaggac cccaggaccc tgcagatgct gcagagcctc ctggaaagcc aacgggatca    3420
   gaggaccatc agcatggccg agccacacag ctcccaggag ctctggatac aagctcccca    3480
   gggacccagc atccctttgc ccctgagaca ccaatccctg gagcatcctg gagcatctcc    3540
   cctaccaccc ccggggggct gccttggggc tggactcaga cacctacgcc agtccctgag    3600
   gacaaagggc aacctggaga agacctgaga catcccggca ccagcctccc tgctgacctg    3660
   cccgggcggc cgcccgagcc ctatagtgag tctgattag                           3699
<210> 18
   <211> 1232
   <212> PRT
   <213> homo sapiens
<400> 18
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465                 470                 475                 480
   Gln Cys Arg Phe Asp Phe Gly Ser Gly Tyr Gln Thr Cys Leu Ala Phe
                   485                 490                 495
   Arg Thr Phe Glu Pro Cys Lys Gln Leu Trp Cys Ser His Pro Asp Asn
               500                 505                 510
   Pro Tyr Phe Cys Lys Thr Lys Lys Gly Pro Pro Leu Asp Gly Thr Glu
           515                 520                 525
   Cys Ala Pro Gly Lys Trp Cys Phe Lys Gly His Cys Ile Trp Lys Ser
       530                 535                 540
   Pro Glu Gln Thr Tyr Gly Gln Asp Gly Gly Trp Ser Ser Trp Thr Lys
   545                 550                 555                 560
   Phe Gly Ser Cys Ser Arg Ser Cys Gly Gly Gly Val Arg Ser Arg Ser
                   565                 570                 575
   Arg Ser Cys Asn Asn Pro Ser Pro Ala Tyr Gly Gly Arg Pro Cys Leu
               580                 585                 590
   Gly Pro Met Phe Glu Tyr Gln Val Cys Asn Ser Glu Glu Cys Pro Gly
           595                 600                 605
   Thr Tyr Glu Asp Phe Arg Ala Gln Gln Cys Ala Lys Arg Asn Ser Tyr
       610                 615                 620
   Tyr Val His Gln Asn Ala Lys His Ser Trp Val Pro Tyr Glu Pro Asp
   625                 630                 635                 640
   Asp Asp Ala Gln Lys Cys Glu Leu Ile Cys Gln Ser Ala Asp Thr Gly
                   645                 650                 655
   Asp Val Val Phe Met Asn Gln Val Val His Asp Gly Thr Arg Cys Ser
               660                 665                 670
   Tyr Arg Asp Pro Tyr Ser Val Cys Ala Arg Gly Glu Cys Val Pro Val
           675                 680                 685
   Gly Cys Asp Lys Glu Val Gly Ser Met Lys Ala Asp Asp Lys Cys Gly
       690                 695                 700
   Val Cys Gly Gly Asp Asn Ser His Cys Arg Thr Val Lys Gly Thr Leu
   705                 710                 715                 720
   Gly Lys Ala Ser Lys Gln Ala Gly Ala Leu Lys Leu Val Gln Ile Pro
                   725                 730                 735
   Ala Gly Ala Arg His Ile Gln Ile Glu Ala Leu Glu Lys Ser Pro His
               740                 745                 750
   Arg Ser Val Val Lys Asn Gln Val Thr Gly Ser Phe Ile Leu Asn Pro
           755                 760                 765
   Lys Gly Lys Glu Ala Thr Ser Arg Thr Phe Thr Ala Met Gly Leu Glu
       770                 775                 780
   Trp Glu Asp Ala Val Glu Asp Ala Lys Glu Ser Leu Lys Thr Ser Gly
   785                 790                 795                 800
   Pro Leu Pro Glu Ala Ile Ala Ile Leu Ala Leu Pro Pro Thr Glu Gly
                   805                 810                 815
   Gly Pro Arg Ser Ser Leu Ala Tyr Lys Tyr Val Ile His Glu Asp Leu
               820                 825                 830
   Leu Pro Leu Ile Gly Ser Asn Asn Val Leu Leu Glu Glu Met Asp Thr
           835                 840                 845
   Tyr Glu Trp Ala Leu Lys Ser Trp Ala Pro Cys Ser Lys Ala Cys Gly
       850                 855                 860
   Gly Gly Ile Gln Phe Thr Lys Tyr Gly Cys Arg Arg Arg Arg Asp His
   865                 870                 875                 880
   His Met Val Gln Arg His Leu Cys Asp His Lys Lys Arg Pro Lys Pro
                   885                 890                 895
   Ile Arg Arg Arg Cys Asn Gln His Pro Cys Ser Gln Pro Val Trp Val
               900                 905                 910
   Thr Glu Glu Trp Gly Ala Cys Ser Arg Ser Cys Gly Lys Leu Gly Val
           915                 920                 925
   Gln Thr Arg Gly Ile Gln Cys Leu Leu Pro Leu Ser Asn Gly Thr His
       930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
   980 985 990
   Val Val Cys Arg Thr Asn Ala Asn Ser Leu Gly His Cys Glu Gly Asp
   995 1000 1005
   Arg Pro Asp Thr Val Gln Val Cys Ser Leu Pro Ala Cys Gly Gly Asn
   1010 1015 1020
   His Gln Asn Ser Thr Val Arg Ala Asp Val Trp Glu Leu Gly Thr Pro
   1025 1030 1035 1040
   Glu Gly Gln Trp Val Pro Gln Ser Gly Pro Leu His Pro Ile Asn Lys
   1045 1050 1055
   Ile Ser Ser Thr Glu Pro Cys Thr Gly Asp Arg Ser Val Phe Cys Gln
   1060 1065 1070
   Met Glu Val Leu Asp Arg Tyr Cys Ser Ile Pro Gly Tyr His Arg Leu
   1075 1080 1085
   Cys Cys Val Ser Cys Ile Lys Lys Ala Ser Gly Pro Asn Pro Gly Pro
   1090 1095 1100
   Asp Pro Gly Pro Thr Ser Leu Pro Pro Phe Ser Thr Pro Gly Ser Pro
   1105 1110 1115 1120
   Leu Pro Gly Pro Gln Asp Pro Ala Asp Ala Ala Glu Pro Pro Gly Lys
   1125 1130 1135
   Pro Thr Gly Ser Glu Asp His Gln His Gly Arg Ala Thr Gln Leu Pro
   1140 1145 1150
   Gly Ala Leu Asp Thr Ser Ser Pro Gly Thr Gln His Pro Phe Ala Pro
   1155 1160 1165
   Glu Thr Pro Ile Pro Gly Ala Ser Trp Ser Ile Ser Pro Thr Thr Pro
   1170 1175 1180
   Gly Gly Leu Pro Trp Gly Trp Thr Gln Thr Pro Thr Pro Val Pro Glu
   1185 1190 1195 1200
   Asp Lys Gly Gln Pro Gly Glu Asp Leu Arg His Pro Gly Thr Ser Leu
   1205 1210 1215
   Pro Ala Asp Leu Pro Gly Arg Pro Pro Glu Pro Tyr Ser Glu Ser Asp
   1220 1225 1230
<210> 19
   <211> 3759
   <212> DNA
   <213> homo sapiens
<400> 19
   atggctccac tccgcgcgct gctgtcctac ctgctgcctt tgcactgtgc gctctgcrcc     60
   gccgcgggca gccggacccc agagctgcac ctctctggaa agctcagtga ctatggtgtg    120
   acagtgccct gcagcacaga ctttcgggga cgcttcctct cccacgtggt gtctggccca    180
   gcagcagcct ctgcagggag catggtagtg gacacgccac ccacactacc acgacactcc    240
   agtcacctcc gggtggctcg cagccctctg cacccaggag ggaccctgtg gcctggcagg    300
   gtggggcgcc actccctcta cttcaatgtc actgttttcg ggaaggaact gcacttgcgc    360
   ctgcggccca atcggaggtt ggtagtgcca ggatcctcag tggagtggca ggaggatttt    420
   cgggagctgt tccggcagcc cttacggcag gagtgtgtgt acactggagg tgtcactgga    480
   atgcctgggg cagctgttgc catcagcaac tgtgacggat tggcgggcct catccgcaca    540
   gacagcaccg acttcttcat tgagcctctg gagcggggcc agcaggagaa ggaggccagc    600
   gggaggacac atgtggtgta ccgccgggag gccgtccagc aggagtgggc agaacctgac    660
   ggggacctgc acaatgaagc ctttggcctg ggagaccttc ccaacctgct gggcctggtg    720
   ggggaccagc tgggcgacac agagcggaag cggcggcatg ccaagccagg cagctacagc    780
   atcgaggtgc tgctggtggt ggacgactcg gtggttcgct tccatggcaa ggagcatgtg    840
   cagaactatg tcctcaccct catgaatatc gtagatgaga tttaccacga tgagtccctg    900
   ggggttcata taaatattgc cctcgtccgc ttgatcatgg ttggctaccg acagtccctg    960
   agcctgatcg agcgcgggaa cccctcacgc agcctggagc aggtgtgtcg ctgggcacac   1020
   tcccagcagc gccaggaccc cagccacgct gagcaccatg accacgttgt gttcctcacc    1080
   cggcaggact ttgggccctc agggtatgca cccgtcactg gcatgtgtca ccccctgagg    1140
   agctgtgccc tcaaccatga ggatggcttc tcctcagcct tcgtgatagc tcatgagacc    1200
   ggccacgtgc tcggcatgga gcatgacggt caggggaatg gctgtgcaga tgagaccagc    1260
   ctgggcagcg tcatggcgcc cctggtgcag gctgccttcc accgcttcca ttggtcccgc    1320
   tgcagcaagc tggagctcag ccgctacctc ccctcctacg actgcctcct cgatgacccc    1380
   tttgatcctg cctggcccca gcccccagag ctgcctggga tcaactactc aatggatgag    1440
   cagtgccgct ttgactttgg cagtggctac cagacctgct tggcattcag gacctttgag    1500
   ccctgcaagc agctgtggtg cagccatcct gacaaccmgt ayttctgcaa gaccaagaag    1560
   gggcccccgc tggatgggac tgagtgtgca cccggcaagt ggtgcttcaa aggtcactgc    1620
   atctggaagt cgccggagca gacatatggc caggatggag gctggagctc ctggaccaag    1680
   tttgggtcat gttcgcggtc atgtgggggc ggggtgcgat cccgcagccg gagctgcaac    1740
   aacccctccc cagcctatgg aggccgccyg tgcttagggc ccatgttcga gtaccaggtc    1800
   tgcaacagcg aggagtgccc tgggacctac gaggacttcc gggcccagca gtgtgccaag    1860
   cgcaactcst actatgtgca ccagaatgcc aagcacagst gggtgcccta cgagcctgac    1920
   gatgacgccc agaagtgtga gctgatctgc cagtcggcgg acacrgggga cgtggtgttc    1980
   atgaaccagg tggttcacga tgggacacgc tgcagctacc gggacccata cagcgtctgt    2040
   gcgcgtggcg agtgtgtgcc tgtcggctgt gacaaggagg tggggtccat gaaggcggat    2100
   gacaagtgtg gagtctgcgg gggtgacaac tcccactgca ggactgtgaa ggggacgctg    2160
   ggcaaggcct ccaagcaggc aggagctctc aagctggtgc agatcccagc aggtgccagg    2220
   cacatccaga ttgaggcact ggagaagtcc ccccaccgsw ywgtggtgaa gaaccaggtc    2280
   accggcagct tcatcctcaa ccccaagggc aaggaagcca caagccggac cttcaccgcc    2340
   atgggcctgg agtgggagga tgcggtggag gatgccaagg aaagcctcaa gaccagcggg    2400
   cccctgcctg aagccattgc catcctggct ctccccccaa ctgagggtgg cccccgcagc    2460
   agcctggcct acaagtacgt catccatgag gacctgctgc cccttatcgg gagcaacaat    2520
   gtgctcctgg aggagatgga cacctatgag tgggcgctca agagctgggc cccctgcagc    2580
   aaggcctgtg gaggagggat ccagttcacc aaatacggct gccggcgcag acgagaccac    2640
   cacatggtgc agcgacacct gtgtgaccac aagaagaggc ccaagcccat ccgccggcgc    2700
   tgcaaccagc acccgtgctc tcagcctgtg tgggtgacgg aggagtgggg tgcctgcagc    2760
   cggagctgtg ggaagctggg ggtgcagaca cgggggatac agtgcctgct gcccctctcc    2820
   aatggaaccc acaaggtcat gccggccaaa gcctgcgccg gggaccggcc tgaggcccga    2880
   cggccctgtc tccgagtgcc ctgcccagcc cagtggaggc tgggagcctg gtcccagtgc    2940
   tctgccacct gtggagaggg catccagcag cggcaggtgg tgtgcaggac caacgccaac    3000
   agcctcgggc attgcgaggg ggataggcca gacactgtcc aggtctgcar cctgcccgcc    3060
   tgtggaggaa atcaccagaa ctccacggtg agggccgatg tctgggaact tgggacgcca    3120
   gaggggcagt gggtgccaca atctgraccc ctacatccca ttaacaagat atcatcaatg    3180
   tgtgcagcgg agccctgcac gggagacagg tctgtcttct gccagatgga agtgctcgat    3240
   cgctactgct ccattcccgg ctaccaccgg ctctgctgtg tgtcctgcat caagaaggcc    3300
   tcgggcccca accctggccc agaccctggc ccaacctcac tgcccccctt ctccactcct    3360
   ggaagcccct taccaggacc ccaggaccct gcagatgctg cagagcctcc tggaaagcca    3420
   acgggatcag aggaccatca gcatggccga gccacacagc tcccaggagc tctggataca    3480
   agctccccag ggacccagca tccctttgcc cctgagacac caatccctgg agcatcctgg    3540
   agcatctccc ctaccacccc cggggggctg ccttggggct ggactcagac acctacgcca    3600
   gtccctgagg acaaagggca acctggagaa gacctgagac atcccggcac cagcctccct    3660
   gctgacctgc ccgggcggcc gcccgagccc tgccatccca ctggcacgtt tacactctgt    3720
   gtactgcccc gtgactccca gctcagagga cacacatag                            3759
<210> 20
   <211> 1252
   <212> PRT
   <213> homo sapiens
<400> 20
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
                   20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385                 390                 395                 400
   Gly His Val Leu Gly Met Glu His Asp Gly Gln Gly Asn Gly Cys Ala
                   405                 410                 415
   Asp Glu Thr Ser Leu Gly Ser Val Met Ala Pro Leu Val Gln Ala Ala
               420                 425                 430
   Phe His Arg Phe His Trp Ser Arg Cys Ser Lys Leu Glu Leu Ser Arg
           435                 440                 445
   Tyr Leu Pro Ser Tyr Asp Cys Leu Leu Asp Asp Pro Phe Asp Pro Ala
       450                 455                 460
   Trp Pro Gln Pro Pro Glu Leu Pro Gly Ile Asn Tyr Ser Met Asp Glu
   465 470 475 480
   Gln Cys Arg Phe Asp Phe Gly Ser Gly Tyr Gln Thr Cys Leu Ala Phe
   485 490 495
   Arg Thr Phe Glu Pro Cys Lys Gln Leu Trp Cys Ser His Pro Asp Asn
   500 505 510
   Pro Tyr Phe Cys Lys Thr Lys Lys Gly Pro Pro Leu Asp Gly Thr Glu
   515 520 525
   Cys Ala Pro Gly Lys Trp Cys Phe Lys Gly His Cys Ile Trp Lys Ser
   530 535 540
   Pro Glu Gln Thr Tyr Gly Gln Asp Gly Gly Trp Ser Ser Trp Thr Lys
   545 550 555 560
   Phe Gly Ser Cys Ser Arg Ser Cys Gly Gly Gly Val Arg Ser Arg Ser
   565 570 575
   Arg Ser Cys Asn Asn Pro Ser Pro Ala Tyr Gly Gly Arg Pro Cys Leu
   580 585 590
   Gly Pro Met Phe Glu Tyr Gln Val Cys Asn Ser Glu Glu Cys Pro Gly
   595 600 605
   Thr Tyr Glu Asp Phe Arg Ala Gln Gln Cys Ala Lys Arg Asn Ser Tyr
   610 615 620
   Tyr Val His Gln Asn Ala Lys His Ser Trp Val Pro Tyr Glu Pro Asp
   625 630 635 640
   Asp Asp Ala Gln Lys Cys Glu Leu Ile Cys Gln Ser Ala Asp Thr Gly
   645 650 655
   Asp Val Val Phe Met Asn Gln Val Val His Asp Gly Thr Arg Cys Ser
   660 665 670
   Tyr Arg Asp Pro Tyr Ser Val Cys Ala Arg Gly Glu Cys Val Pro Val
   675 680 685
   Gly Cys Asp Lys Glu Val Gly Ser Met Lys Ala Asp Asp Lys Cys Gly
   690 695 700
   Val Cys Gly Gly Asp Asn Ser His Cys Arg Thr Val Lys Gly Thr Leu
   705 710 715 720
   Gly Lys Ala Ser Lys Gln Ala Gly Ala Leu Lys Leu Val Gln Ile Pro
   725 730 735
   Ala Gly Ala Arg His Ile Gln Ile Glu Ala Leu Glu Lys Ser Pro His
   740 745 750
   Arg Ser Val Val Lys Asn Gln Val Thr Gly Ser Phe Ile Leu Asn Pro
   755 760 765
   Lys Gly Lys Glu Ala Thr Ser Arg Thr Phe Thr Ala Met Gly Leu Glu
   770 775 780
   Trp Glu Asp Ala Val Glu Asp Ala Lys Glu Ser Leu Lys Thr Ser Gly
   785 790 795 800
   Pro Leu Pro Glu Ala Ile Ala Ile Leu Ala Leu Pro Pro Thr Glu Gly
   805 810 815
   Gly Pro Arg Ser Ser Leu Ala Tyr Lys Tyr Val Ile His Glu Asp Leu
   820 825 830
   Leu Pro Leu Ile Gly Ser Asn Asn Val Leu Leu Glu Glu Met Asp Thr
   835 840 845
   Tyr Glu Trp Ala Leu Lys Ser Trp Ala Pro Cys Ser Lys Ala Cys Gly
   850 855 860
   Gly Gly Ile Gln Phe Thr Lys Tyr Gly Cys Arg Arg Arg Arg Asp His
   865 870 875 880
   His Met Val Gln Arg His Leu Cys Asp His Lys Lys Arg Pro Lys Pro
   885 890 895
   Ile Arg Arg Arg Cys Asn Gln His Pro Cys Ser Gln Pro Val Trp Val
   900 905 910
   Thr Glu Glu Trp Gly Ala Cys Ser Arg Ser Cys Gly Lys Leu Gly Val
                   915                 920                 925
   Gln Thr Arg Gly Ile Gln Cys Leu Leu Pro Leu Ser Asn Gly Thr His
               930                 935                 940
   Lys Val Met Pro Ala Lys Ala Cys Ala Gly Asp Arg Pro Glu Ala Arg
   945                 950                 955                 960
   Arg Pro Cys Leu Arg Val Pro Cys Pro Ala Gln Trp Arg Leu Gly Ala
                   965                 970                 975
   Trp Ser Gln Cys Ser Ala Thr Cys Gly Glu Gly Ile Gln Gln Arg Gln
               980                 985                 990
   Val Val Cys Arg Thr Asn Ala Asn Ser Leu Gly His Cys Glu Gly Asp
           995                 1000                1005
   Arg Pro Asp Thr Val Gln Val Cys Ser Leu Pro Ala Cys Gly Gly Asn
       1010                1015                1020
   His Gln Asn Ser Thr Val Arg Ala Asp Val Trp Glu Leu Gly Thr Pro
   1025                1030                1035                1040
   Glu Gly Gln Trp Val Pro Gln Ser Gly Pro Leu His Pro Ile Asn Lys
                   1045                1050                1055
   Ile Ser Ser Met Cys Ala Ala Glu Pro Cys Thr Gly Asp Arg Ser Val
               1060                1065                1070
   Phe Cys Gln Met Glu Val Leu Asp Arg Tyr Cys Ser Ile Pro Gly Tyr
           1075                1080                1085
   His Arg Leu Cys Cys Val Ser Cys Ile Lys Lys Ala Ser Gly Pro Asn
       1090                1095                1100
   Pro Gly Pro Asp Pro Gly Pro Thr Ser Leu Pro Pro Phe Ser Thr Pro
   1105                1110                1115                1120
   Gly Ser Pro Leu Pro Gly Pro Gln Asp Pro Ala Asp Ala Ala Glu Pro
                   1125                1130                1135
   Pro Gly Lys Pro Thr Gly Ser Glu Asp His Gln His Gly Arg Ala Thr
               1140                1145                1150
   Gln Leu Pro Gly Ala Leu Asp Thr Ser Ser Pro Gly Thr Gln His Pro
           1155                1160                1165
   Phe Ala Pro Glu Thr Pro Ile Pro Gly Ala Ser Trp Ser Ile Ser Pro
       1170                1175                1180
   Thr Thr Pro Gly Gly Leu Pro Trp Gly Trp Thr Gln Thr Pro Thr Pro
   1185                1190                1195                1200
   Val Pro Glu Asp Lys Gly Gln Pro Gly Glu Asp Leu Arg His Pro Gly
                   1205                1210                1215
   Thr Ser Leu Pro Ala Asp Leu Pro Gly Arg Pro Pro Glu Pro Cys His
               1220                1225                1230
   Pro Thr Gly Thr Phe Thr Leu Cys Val Leu Pro Arg Asp Ser Gln Leu
           1235                1240                1245
   Arg Gly His Thr
       1250
<210> 21
   <211> 3750
   <212> DNA
   <213> homo sapiens
<400> 21
   atggctccac tccgcgcgct gctgtcctac ctgctgcctt tgcactgtgc gctctgcrcc   60
   gccgcgggca gccggacccc agagctgcac ctctctggaa agctcagtga ctatggtgtg  120
   acagtgccct gcagcacaga ctttcgggga cgcttcctct cccacgtggt gtctggccca  180
   gcagcagcct ctgcagggag catggtagtg gacacgccac ccacactacc acgacactcc  240
   agtcacctcc gggtggctcg cagccctctg cacccaggag ggaccctgtg gcctggcagg  300
   gtggggcgcc actccctcta cttcaatgtc actgttttcg ggaaggaact gcacttgcgc  360
   ctgcggccca atcggaggtt ggtagtgcca ggatcctcag tggagtggca ggaggatttt 420
   cgggagctgt tccggcagcc cttacggcag gagtgtgtgt acactggagg tgtcactgga 480
   atgcctgggg cagctgttgc catcagcaac tgtgacggat tggcgggcct catccgcaca 540
   gacagcaccg acttcttcat tgagcctctg gagcggggcc agcaggagaa ggaggccagc 600
   gggaggacac atgtggtgta ccgccgggag gccgtccagc aggagtgggc agaacctgac 660
   ggggacctgc acaatgaagc ctttggcctg ggagaccttc ccaacctgct gggcctggtg 720
   ggggaccagc tgggcgacac agagcggaag cggcggcatg ccaagccagg cagctacagc 780
   atcgaggtgc tgctggtggt ggacgactcg gtggttcgct tccatggcaa ggagcatgtg 840
   cagaactatg tcctcaccct catgaatatc gtagatgaga tttaccacga tgagtccctg 900
   ggggttcata taaatattgc cctcgtccgc ttgatcatgg ttggctaccg acagtccctg 960
   agcctgatcg agcgcgggaa cccctcacgc agcctggagc aggtgtgtcg ctgggcacac 1020
   tcccagcagc gccaggaccc cagccacgct gagcaccatg accacgttgt gttcctcacc 1080
   cggcaggact ttgggccctc agggtatgca cccgtcactg gcatgtgtca ccccctgagg 1140
   agctgtgccc tcaaccatga ggatggcttc tcctcagcct tcgtgatagc tcatgagacc 1200
   ggccacgtgc tcggcatgga gcatgacggt caggggaatg gctgtgcaga tgagaccagc 1260
   ctgggcagcg tcatggcgcc cctggtgcag gctgccttcc accgcttcca ttggtcccgc 1320
   tgcagcaagc tggagctcag ccgctacctc ccctcctacg actgcctcct cgatgacccc 1380
   tttgatcctg cctggcccca gcccccagag ctgcctggga tcaactactc aatggatgag 1440
   cagtgccgct ttgactttgg cagtggctac cagacctgct tggcattcag gacctttgag 1500
   ccctgcaagc agctgtggtg cagccatcct gacaaccmgt ayttctgcaa gaccaagaag 1560
   gggcccccgc tggatgggac tgagtgtgca cccggcaagt ggtgcttcaa aggtcactgc 1620
   atctggaagt cgccggagca gacatatggc caggatggag gctggagctc ctggaccaag 1680
   tttgggtcat gttcgcggtc atgtgggggc ggggtgcgat cccgcagccg gagctgcaac 1740
   aacccctccc cagcctatgg aggccgccyg tgcttagggc ccatgttcga gtaccaggtc 1800
   tgcaacagcg aggagtgccc tgggacctac gaggacttcc gggcccagca gtgtgccaag 1860
   cgcaactcst actatgtgca ccagaatgcc aagcacagst gggtgcccta cgagcctgac 1920
   gatgacgccc agaagtgtga gctgatctgc cagtcggcgg acacrgggga cgtggtgttc 1980
   atgaaccagg tggttcacga tgggacacgc tgcagctacc gggacccata cagcgtctgt 2040
   gcgcgtggcg agtgtgtgcc tgtcggctgt gacaaggagg tggggtccat gaaggcggat 2100
   gacaagtgtg gagtctgcgg gggtgacaac tcccactgca ggactgtgaa ggggacgctg 2160
   ggcaaggcct ccaagcaggc aggagctctc aagctggtgc agatcccagc aggtgccagg 2220
   cacatccaga ttgaggcact ggagaagtcc ccccaccgsw ywgtggtgaa gaaccaggtc 2280
   accggcagct tcatcctcaa ccccaagggc aaggaagcca caagccggac cttcaccgcc 2340
   atgggcctgg agtgggagga tgcggtggag gatgccaagg aaagcctcaa gaccagcggg 2400
   cccctgcctg aagccattgc catcctggct ctccccccaa ctgagggtgg cccccgcagc 2460
   agcctggcct acaagtacgt catccatgag gacctgctgc cccttatcgg gagcaacaat 2520
   gtgctcctgg aggagatgga cacctatgag tgggcgctca agagctgggc cccctgcagc 2580
   aaggcctgtg gaggagggat ccagttcacc aaatacggct gccggcgcag acgagaccac 2640
   cacatggtgc agcgacacct gtgtgaccac aagaagaggc ccaagcccat ccgccggcgc 2700
   tgcaaccagc acccgtgctc tcagcctgtg tgggtgacgg aggagtgggg tgcctgcagc 2760
   cggagctgtg ggaagctggg ggtgcagaca cgggggatac agtgcctgct gcccctctcc 2820
   aatggaaccc acaaggtcat gccggccaaa gcctgcgccg gggaccggcc tgaggcccga 2880
   cggccctgtc tccgagtgcc ctgcccagcc cagtggaggc tgggagcctg gtcccagtgc 2940
   tctgccacct gtggagaggg catccagcag cggcaggtgg tgtgcaggac caacgccaac 3000
   agcctcgggc attgcgaggg ggataggcca gacactgtcc aggtctgcar cctgcccgcc 3060
   tgtggaggaa atcaccagaa ctccacggtg agggccgatg tctgggaact tgggacgcca 3120
   gaggggcagt gggtgccaca atctgraccc ctacatccca ttaacaagat atcatcaacg 3180
   gagccctgca cgggagacag gtctgtcttc tgccagatgg aagtgctcga tcgctactgc 3240
   tccattcccg gctaccaccg gctctgctgt gtgtcctgca tcaagaaggc ctcgggcccc 3300
   aaccctggcc cagaccctgg cccaacctca ctgcccccct tctccactcc tggaagcccc 3360
   ttaccaggac cccaggaccc tgcagatgct gcagagcctc ctggaaagcc aacgggatca 3420
   gaggaccatc agcatggccg agccacacag ctcccaggag ctctggatac aagctcccca 3480
   gggacccagc atccctttgc ccctgagaca ccaatccctg gagcatcctg gagcatctcc 3540
   cctaccaccc ccggggggct gccttggggc tggactcaga cacctacgcc agtccctgag 3600
   gacaaagggc aacctggaga agacctgaga catcccggca ccagcctccc tgctgacctg 3660
   cccgggcggc cgcccgagcc ctgccatccc actggcacgt ttacactctg tgtactgccc 3720
   cgtgactccc agctcagagg acacacatag 3750
<210> 22
   <211> 1249
   <212> PRT
   <213> homo sapiens
<400> 22
   Met Ala Pro Leu Arg Ala Leu Leu Ser Tyr Leu Leu Pro Leu His Cys
    1               5                  10                  15
   Ala Leu Cys Thr Ala Ala Gly Ser Arg Thr Pro Glu Leu His Leu Ser
               20                  25                  30
   Gly Lys Leu Ser Asp Tyr Gly Val Thr Val Pro Cys Ser Thr Asp Phe
           35                  40                  45
   Arg Gly Arg Phe Leu Ser His Val Val Ser Gly Pro Ala Ala Ala Ser
       50                  55                  60
   Ala Gly Ser Met Val Val Asp Thr Pro Pro Thr Leu Pro Arg His Ser
   65                  70                  75                  80
   Ser His Leu Arg Val Ala Arg Ser Pro Leu His Pro Gly Gly Thr Leu
                   85                  90                  95
   Trp Pro Gly Arg Val Gly Arg His Ser Leu Tyr Phe Asn Val Thr Val
               100                 105                 110
   Phe Gly Lys Glu Leu His Leu Arg Leu Arg Pro Asn Arg Arg Leu Val
           115                 120                 125
   Val Pro Gly Ser Ser Val Glu Trp Gln Glu Asp Phe Arg Glu Leu Phe
       130                 135                 140
   Arg Gln Pro Leu Arg Gln Glu Cys Val Tyr Thr Gly Gly Val Thr Gly
   145                 150                 155                 160
   Met Pro Gly Ala Ala Val Ala Ile Ser Asn Cys Asp Gly Leu Ala Gly
                   165                 170                 175
   Leu Ile Arg Thr Asp Ser Thr Asp Phe Phe Ile Glu Pro Leu Glu Arg
               180                 185                 190
   Gly Gln Gln Glu Lys Glu Ala Ser Gly Arg Thr His Val Val Tyr Arg
           195                 200                 205
   Arg Glu Ala Val Gln Gln Glu Trp Ala Glu Pro Asp Gly Asp Leu His
       210                 215                 220
   Asn Glu Ala Phe Gly Leu Gly Asp Leu Pro Asn Leu Leu Gly Leu Val
   225                 230                 235                 240
   Gly Asp Gln Leu Gly Asp Thr Glu Arg Lys Arg Arg His Ala Lys Pro
                   245                 250                 255
   Gly Ser Tyr Ser Ile Glu Val Leu Leu Val Val Asp Asp Ser Val Val
               260                 265                 270
   Arg Phe His Gly Lys Glu His Val Gln Asn Tyr Val Leu Thr Leu Met
           275                 280                 285
   Asn Ile Val Asp Glu Ile Tyr His Asp Glu Ser Leu Gly Val His Ile
       290                 295                 300
   Asn Ile Ala Leu Val Arg Leu Ile Met Val Gly Tyr Arg Gln Ser Leu
   305                 310                 315                 320
   Ser Leu Ile Glu Arg Gly Asn Pro Ser Arg Ser Leu Glu Gln Val Cys
                   325                 330                 335
   Arg Trp Ala His Ser Gln Gln Arg Gln Asp Pro Ser His Ala Glu His
               340                 345                 350
   His Asp His Val Val Phe Leu Thr Arg Gln Asp Phe Gly Pro Ser Gly
           355                 360                 365
   Tyr Ala Pro Val Thr Gly Met Cys His Pro Leu Arg Ser Cys Ala Leu
       370                 375                 380
   Asn His Glu Asp Gly Phe Ser Ser Ala Phe Val Ile Ala His Glu Thr
   385 390 395 400
   Gly His Val Leu Gly Met Glu His Asp Gly Gln Gly Asn Gly Cys Ala
   405 410 415
   Asp Glu Thr Ser Leu Gly Ser Val Met Ala Pro Leu Val Gln Ala Ala
   420 425 430
   Phe His Arg Phe His Trp Ser Arg Cys Ser Lys Leu Glu Leu Ser Arg
   435 440 445
   Tyr Leu Pro Ser Tyr Asp Cys Leu Leu Asp Asp Pro Phe Asp Pro Ala
   450 455 460
   Trp Pro Gln Pro Pro Glu Leu Pro Gly Ile Asn Tyr Ser Met Asp Glu
   465 470 475 480
   Gln Cys Arg Phe Asp Phe Gly Ser Gly Tyr Gln Thr Cys Leu Ala Phe
   485 490 495
   Arg Thr Phe Glu Pro Cys Lys Gln Leu Trp Cys Ser His Pro Asp Asn
   500 505 510
   Pro Tyr Phe Cys Lys Thr Lys Lys Gly Pro Pro Leu Asp Gly Thr Glu
   515 520 525
   Cys Ala Pro Gly Lys Trp Cys Phe Lys Gly His Cys Ile Trp Lys Ser
   530 535 540
   Pro Glu Gln Thr Tyr Gly Gln Asp Gly Gly Trp Ser Ser Trp Thr Lys
   545 550 555 560
   Phe Gly Ser Cys Ser Arg Ser Cys Gly Gly Gly Val Arg Ser Arg Ser
   565 570 575
   Arg Ser Cys Asn Asn Pro Ser Pro Ala Tyr Gly Gly Arg Pro Cys Leu
   580 585 590
   Gly Pro Met Phe Glu Tyr Gln Val Cys Asn Ser Glu Glu Cys Pro Gly
   595 600 605
   Thr Tyr Glu Asp Phe Arg Ala Gln Gln Cys Ala Lys Arg Asn Ser Tyr
   610 615 620
   Tyr Val His Gln Asn Ala Lys His Ser Trp Val Pro Tyr Glu Pro Asp
   625 630 635 640
   Asp Asp Ala Gln Lys Cys Glu Leu Ile Cys Gln Ser Ala Asp Thr Gly
   645 650 655
   Asp Val Val Phe Met Asn Gln Val Val His Asp Gly Thr Arg Cys Ser
   660 665 670
   Tyr Arg Asp Pro Tyr Ser Val Cys Ala Arg Gly Glu Cys Val Pro Val
   675 680 685
   Gly Cys Asp Lys Glu Val Gly Ser Met Lys Ala Asp Asp Lys Cys Gly
   690 695 700
   Val Cys Gly Gly Asp Asn Ser His Cys Arg Thr Val Lys Gly Thr Leu
   705 710 715 720
   Gly Lys Ala Ser Lys Gln Ala Gly Ala Leu Lys Leu Val Gln Ile Pro
   725 730 735
   Ala Gly Ala Arg His Ile Gln Ile Glu Ala Leu Glu Lys Ser Pro His
   740 745 750
   Arg Ser Val Val Lys Asn Gln Val Thr Gly Ser Phe Ile Leu Asn Pro
   755 760 765
   Lys Gly Lys Glu Ala Thr Ser Arg Thr Phe Thr Ala Met Gly Leu Glu
   770 775 780
   Trp Glu Asp Ala Val Glu Asp Ala Lys Glu Ser Leu Lys Thr Ser Gly
   785 790 795 800
   Pro Leu Pro Glu Ala Ile Ala Ile Leu Ala Leu Pro Pro Thr Glu Gly
   805 810 815
   Gly Pro Arg Ser Ser Leu Ala Tyr Lys Tyr Val Ile His Glu Asp Leu
   820 825 830
   Leu Pro Leu Ile Gly Ser Asn Asn Val Leu Leu Glu Glu Met Asp Thr
   835 840 845
   Tyr Glu Trp Ala Leu Lys Ser Trp Ala Pro Cys Ser Lys Ala Cys Gly
   850 855 860
   Gly Gly Ile Gln Phe Thr Lys Tyr Gly Cys Arg Arg Arg Arg Asp His
   865 870 875 880
   His Met Val Gln Arg His Leu Cys Asp His Lys Lys Arg Pro Lys Pro
   885 890 895
   Ile Arg Arg Arg Cys Asn Gln His Pro Cys Ser Gln Pro Val Trp Val
   900 905 910
   Thr Glu Glu Trp Gly Ala Cys Ser Arg Ser Cys Gly Lys Leu Gly Val
   915 920 925
   Gln Thr Arg Gly Ile Gln Cys Leu Leu Pro Leu Ser Asn Gly Thr His
   930 935 940
   Lys Val Met Pro Ala Lys Ala Cys Ala Gly Asp Arg Pro Glu Ala Arg
   945 950 955 960
   Arg Pro Cys Leu Arg Val Pro Cys Pro Ala Gln Trp Arg Leu Gly Ala
   965 970 975
   Trp Ser Gln Cys Ser Ala Thr Cys Gly Glu Gly Ile Gln Gln Arg Gln
   980 985 990
   Val Val Cys Arg Thr Asn Ala Asn Ser Leu Gly His Cys Glu Gly Asp
   995 1000 1005
   Arg Pro Asp Thr Val Gln Val Cys Ser Leu Pro Ala Cys Gly Gly Asn
   1010 1015 1020
   His Gln Asn Ser Thr Val Arg Ala Asp Val Trp Glu Leu Gly Thr Pro
   1025 1030 1035 1040
   Glu Gly Gln Trp Val Pro Gln Ser Gly Pro Leu His Pro Ile Asn Lys
   1045 1050 1055
   Ile Ser Ser Thr Glu Pro Cys Thr Gly Asp Arg Ser Val Phe Cys Gln
   1060 1065 1070
   Met Glu Val Leu Asp Arg Tyr Cys Ser Ile Pro Gly Tyr His Arg Leu
   1075 1080 1085
   Cys Cys Val Ser Cys Ile Lys Lys Ala Ser Gly Pro Asn Pro Gly Pro
   1090 1095 1100
   Asp Pro Gly Pro Thr Ser Leu Pro Pro Phe Ser Thr Pro Gly Ser Pro
   1105 1110 1115 1120
   Leu Pro Gly Pro Gln Asp Pro Ala Asp Ala Ala Glu Pro Pro Gly Lys
   1125 1130 1135
   Pro Thr Gly Ser Glu Asp His Gln His Gly Arg Ala Thr Gln Leu Pro
   1140 1145 1150
   Gly Ala Leu Asp Thr Ser Ser Pro Gly Thr Gln His Pro Phe Ala Pro
   1155 1160 1165
   Glu Thr Pro Ile Pro Gly Ala Ser Trp Ser Ile Ser Pro Thr Thr Pro
   1170 1175 1180
   Gly Gly Leu Pro Trp Gly Trp Thr Gln Thr Pro Thr Pro Val Pro Glu
   1185 1190 1195 1200
   Asp Lys Gly Gln Pro Gly Glu Asp Leu Arg His Pro Gly Thr Ser Leu
   1205 1210 1215
   Pro Ala Asp Leu Pro Gly Arg Pro Pro Glu Pro Cys His Pro Thr Gly
   1220 1225 1230
   Thr Phe Thr Leu Cys Val Leu Pro Arg Asp Ser Gln Leu Arg Gly His
   1235 1240 1245
   Thr
<210> 23
   <211> 4277
   <212> DNA
   <213> homo sapiens
<400> 23
   cagcggtcca ggcggcggcg ccgcgcaggg gacccggagc aggcgggagg gaagcagcta     60
   ggcggggagg cggctgaggc ggcagcggcg gcagccagcc ggtgctccga cagcccgggg    120
   cgcaccctag cctcgccgcc ctcagcctgg gacttgggga tcgggcgctt gcccagcccg    180
   cgtcccagcg cggccacatg gctccactcc gcgcgctgct gtcctacctg ctgcctttgc    240
   actgtgcgct ctgcrccgcc gcgggcagcc ggaccccaga gctgcacctc tctggaaagc    300
   tcagtgacta tggtgtgaca gtgccctgca gcacagactt tcggggacgc ttcctctccc    360
   acgtggtgtc tggcccagca gcagcctctg cagggagcat ggtagtggac acgccaccca    420
   cactaccacg acactccagt cacctccggg tggctcgcag ccctctgcac ccaggaggga    480
   ccctgtggcc tggcagggtg gggcgccact ccctctactt caatgtcact gttttcggga    540
   aggaactgca cttgcgcctg cggcccaatc ggaggttggt agtgccagga tcctcagtgg    600
   agtggcagga ggattttcgg gagctgttcc ggcagccctt acggcaggag tgtgtgtaca    660
   ctggaggtgt cactggaatg cctggggcag ctgttgccat cagcaactgt gacggattgg    720
   cgggcctcat ccgcacagac agcaccgact tcttcattga gcctctggag cggggccagc    780
   aggagaagga ggccagcggg aggacacatg tggtgtaccg ccgggaggcc gtccagcagg    840
   agtgggcaga acctgacggg gacctgcaca atgaagcctt tggcctggga gaccttccca    900
   acctgctggg cctggtgggg gaccagctgg gcgacacaga gcggaagcgg cggcatgcca    960
   agccaggcag ctacagcatc gaggtgctgc tggtggtgga cgactcggtg gttcgcttcc   1020
   atggcaagga gcatgtgcag aactatgtcc tcaccctcat gaatatcgta gatgagattt   1080
   accacgatga gtccctgggg gttcatataa atattgccct cgtccgcttg atcatggttg   1140
   gctaccgaca gtccctgagc ctgatcgagc gcgggaaccc ctcacgcagc ctggagcagg   1200
   tgtgtcgctg ggcacactcc cagcagcgcc aggaccccag ccacgctgag caccatgacc   1260
   acgttgtgtt cctcacccgg caggactttg ggccctcagg gtatgcaccc gtcactggca   1320
   tgtgtcaccc cctgaggagc tgtgccctca accatgagga tggcttctcc tcagccttcg   1380
   tgatagctca tgagaccggc cacgtgctcg gcatggagca tgacggtcag gggaatggct   1440
   gtgcagatga gaccagcctg ggcagcgtca tggcgcccct ggtgcaggct gccttccacc   1500
   gcttccattg gtcccgctgc agcaagctgg agctcagccg ctacctcccc tcctacgact   1560
   gcctcctcga tgaccccttt gatcctgcct ggccccagcc cccagagctg cctgggatca   1620
   actactcaat ggatgagcag tgccgctttg actttggcag tggctaccag acctgcttgg   1680
   cattcaggac ctttgagccc tgcaagcagc tgtggtgcag ccatcctgac aaccmgtayt   1740
   tctgcaagac caagaagggg cccccgctgg atgggactga gtgtgcaccc ggcaagtggt   1800
   gcttcaaagg tcactgcatc tggaagtcgc cggagcagac atatggccag gatggaggct   1860
   ggagctcctg gaccaagttt gggtcatgtt cgcggtcatg tgggggcggg gtgcgatccc  1920
   gcagccggag ctgcaacaac ccctccccag cctatggagg ccgccygtgc ttagggccca   1980
   tgttcgagta ccaggtctgc aacagcgagg agtgccctgg gacctacgag gacttccggg   2040
   cccagcagtg tgccaagcgc aactcstact atgtgcacca gaatgccaag cacagstggg   2100
   tgccctacga gcctgacgat gacgcccaga agtgtgagct gatctgccag tcggcggaca   2160
   crggggacgt ggtgttcatg aaccaggtgg ttcacgatgg gacacgctgc agctaccggg   2220
   acccatacag cgtctgtgcg cgtggcgagt gtgtgcctgt cggctgtgac aaggaggtgg   2280
   ggtccatgaa ggcggatgac aagtgtggag tctgcggggg tgacaactcc cactgcagga   2340
   ctgtgaaggg gacgctgggc aaggcctcca agcaggcagg agctctcaag ctggtgcaga   2400
   tcccagcagg tgccaggcac atccagattg aggcactgga gaagtccccc caccgswywg   2460
   tggtgaagaa ccaggtcacc ggcagcttca tcctcaaccc caagggcaag gaagccacaa   2520
   gccggacctt caccgccatg ggcctggagt gggaggatgc ggtggaggat gccaaggaaa   2580
   gcctcaagac cagcgggccc ctgcctgaag ccattgccat cctggctctc cccccaactg   2640
   agggtggccc ccgcagcagc ctggcctaca agtacgtcat ccatgaggac ctgctgcccc   2700
   ttatcgggag caacaatgtg ctcctggagg agatggacac ctatgagtgg gcgctcaaga   2760
   gctgggcccc ctgcagcaag gcctgtggag gagggatcca gttcaccaaa tacggctgcc   2820
   ggcgcagacg agaccaccac atggtgcagc gacacctgtg tgaccacaag aagaggccca   2880
   agcccatccg ccggcgctgc aaccagcacc cgtgctctca gcctgtgtgg gtgacggagg   2940
   agtggggtgc ctgcagccgg agctgtggga agctgggggt gcagacacgg gggatacagt   3000
   gcctgctgcc cctctccaat ggaacccaca aggtcatgcc ggccaaagcc tgcgccgggg   3060
   accggcctga ggcccgacgg ccctgtctcc gagtgccctg cccagcccag tggaggctgg    3120
   gagcctggtc ccagtgctct gccacctgtg gagagggcat ccagcagcgg caggtggtgt    3180
   gcaggaccaa cgccaacagc ctcgggcatt gcgaggggga taggccagac actgtccagg    3240
   tctgcarcct gcccgcctgt ggaggaaatc accagaactc cacggtgagg gccgatgtct    3300
   gggaacttgg gacgccagag gggcagtggg tgccacaatc tgraccccta catcccatta    3360
   acaagatatc atcaatgtgt gcagcggagc cctgcacggg agacaggtct gtcttctgcc    3420
   agatggaagt gctcgatcgc tactgctcca ttcccggcta ccaccggctc tgctgtgtgt    3480
   cctgcatcaa gaaggcctcg ggccccaacc ctggcccaga ccctggccca acctcactgc    3540
   cccccttctc cactcctgga agccccttac caggacccca ggaccctgca gatgctgcag    3600
   agcctcctgg aaagccaacg ggatcagagg accatcagca tggccgagcc acacagctcc    3660
   caggagctct ggatacaagc tccccaggga cccagcatcc ctttgcccct gagacaccaa    3720
   tccctggagc atcctggagc atctccccta ccacccccgg ggggctgcct tggggctgga    3780
   ctcagacacc tacgccagtc cctgaggaca aagggcaacc tggagaagac ctgagacatc    3840
   ccggcaccag cctccctgct gacctgcccg ggcggccgcc cgagccctgc catcccactg    3900
   gcacgtttac actctgtgta ctgccccgtg actcccagct cagaggacac acatagcagg    3960
   gcaggcgcaa gcacagactt cattttaaat cattcgcctt cttctcgttt ggggctgtga    4020
   tgctctttac cccacaaagc ggggtgggag gaagacaaag atcagggaaa gccctaatcg    4080
   gagatacctc agcaagctgc ccccggcggg actgaccctc tcagggcccc tgttggtctc    4140
   ccctgccaag accagggtca actattgctc cctcctcaca gaccctgggc ctgggcaggt    4200
   ctgaatcccg gctggtctgt agctrgaagc tgtcagggct gcctgccttc ccggaactgt    4260
   gaggacccct gtggagg                                                   4277

## Description

### 1. INTRODUCTION

The present invention relates to the discovery, identification, and characterization of novel human polynucleotides encoding proteins sharing sequence similarity with mammalian proteases. The invention encompasses the described polynucleotides, host cell expression systems, the encoded proteins, fusion proteins, polypeptides and peptides, antibodies to the encoded proteins and peptides, and genetically engineered) non-human animals that either lack or over express the disclosed sequences, antagonists and agonists of the proteins, and other compounds that modulate the expression or activity of the proteins encoded by the disclosed polynucleotides that can be used for diagnosis, drug screening, clinical trial monitoring, the treatment of diseases and disorders, and cosmetic or nutriceutical applications.

### 2. BACKGROUND OF THE INVENTION

Proteases cleave protein substrates as part of degradation, maturation, and secretory pathways within the body. Proteases have been associated with, inter alia, regulating development, diabetes, obesity, infertility, modulating cellular processes, and infectious disease. Proteases are known in the prior art, for example, Bor Luen Trang et al., 1999, FEBS letters, 445 (26): 223-225 described the ADAMTS family of proteases, WO 01 59133 discloses larynx-cancer associated Protein-1 (LarCAP-1) as a putative protease and WO 01 83782 discloses a number of putative human proteases.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with animal proteases and particularly zinc metalloproteases.

The novel human nucleic acid (cDNA) sequences described herein, encode proteins/open reading frames (ORFs) of 451, 297, 486, 1222, 1219, 1216, 1213, 1235, 1232, 1252, and 1249 amino acids in length (see SEQ ID NOS: 2, 4, 6, 8, 10, 12, 14, 16, 18, 20, and 22) respectively). The invention also encompasses agonists and antagonists of the described NHPs, including small molecules, large molecules, mutant NHPs, or portions thereof, that compete with native NHP, peptides, and antibodies, as well as nucleotide sequences that can be used to inhibit the expression of the described NHPs *(e.g.,* antisense and ribozyme molecules, and open reading frame or regulatory sequence replacement constructs) or to enhance the expression of the described NHPs (e.g., expression constructs that place the described polynucleotide under the control of a strong promoter system), and non-human transgenic animals that express a NHP sequence, or "knock-outs" (which can be conditional) that do not express a functional NHP. Knock-out mice can be produced in several ways, one of which involves the use of mouse embryonic stem cells ("ES cells") lines that contain gene trap mutations in a murine homolog of at least one of the described NHPs. When the unique NHP sequences described in SEQ ID NOS:1-23 are "knocked-out" they provide a method of identifying phenotypic expression of the particular gene as well as a method of assigning function to previously unknown genes. In addition, animals in which the unique NHP sequences described in SEQ ID NOS:1-23 are "knocked-out" provide a unique source in which to elicit antibodies to homologous and orthologbus proteins which would have been previously viewed by the immune system as "self" and therefore would have failed to elicit significant antibody responses. To these ends, gene trapped knockout ES cells have been generated in murine homologs of the described NHPs.

Additionally, the unique NHP sequences described in SEQ ID NOS:1-23 are useful for the indentification of protein coding sequence and mapping a unique gene to a particular chromosome. These sequences identify actual, biologically relevant, exon splice junctions as opposed to those that might have been predicted bioinformatically from genomic sequence alone.

Further, the present invention also relates to processes for identifying compounds that modutate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of several NHP ORFs encoding the described NHP amino acid sequences. SEQ ID NO:23 describes an NHP ORF and flanking sequences.

### 5. DETAILED DESCRIPTION OF THE INVENTION

The NHP sequences described for the first time herein are novel proteins that are expressed in, *inter alia*, human cel line, and human spinal cord, lymph node, bone marrow, trachea, mammary gland, skeletal muscle, pericardium, adipose, esophagus, bladder, fetal kidney, and fetal lung cells (SEQ ID NOS:1-23).

The described sequences were compiled from cDNA clones, genomic sequence, and cDNAs derived from human lymph node, thyroid, fetal brain, bone marrow, trachea, kidney, and mammary gland mRNAs (Edge Biosystems, Gaithersburg, MD, and Clontech, Palo Alto, CA). The present invention encompasses the nucleotides presented in the Sequence Listing, host cells expressing such nucleotides, the expression products of such nucleotides, and: (a) nucleotides that encode mammalian homologs of the described genes, including the specifically described NHPs, and NHP products; (b) nucleotides that encode one or more portions of a NHP that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (*e.g*., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF), or a contiguous exon splice junction first described in the Sequence Listing, that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et al., eds., 1989, Current Protocols in Molecular Biology, Vol. I, Green Publishing Associates, Inc., and John Wiley & Sons, Inc., NY, at p. 2.10.3) and encodes a functionally equivalent expression product. Additionally contemplated are any nucleotide sequences that hybridize to the complement of the DNA sequence that encode and express an amino acid sequence presented in the Sequence Listing under moderately stringent conditions, *e.g*., washing in 0.2xSSC/0.1% SDS at 42°C (Ausubel et al., 1989, supra), yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding a NHP ORF, or its functional equivalent, encoded by a polynucleotide sequence that is about 99, 95, 90, or about 85 percent similar or identical to corresponding regions of the nucleotide sequences of the Sequence Listing (as measured by BLAST sequence comparison analysis using, for example, the GCG sequence analysis package using standard default settings).

The invention also includes nucleic acid molecules, preferably DNA molecules, that hybridize to, and are therefore the complements of, the described NHP gene nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-23 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (*i.e*., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-23, or an amino acid sequence encoded thereby. Methods for attaching biopolymers to, or synthesizing biopolymers on, solid support matrices, and conducting binding studies thereon are disclosed in, *inter alia,* U.S. Patent Nos. 5,700,637, 5,556,752, 5,744,305, 4, 631,211, 5,445,934, 5,252,743, 4,713,326, 5,424,186, and 4,689,405.

Addressable arrays comprising sequences first disclosed in SEQ ID NOS: 1-23 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides, and more preferably 25 nucleotides from the sequences first disclosed in SEQ IS NOS:1-23.

For example, a series of the described oligonucleotide sequences, or the complements thereof, can be used in chip format to represent all or a portion of the described secfuences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-26 provides detailed information about transcriptional changes involved in a specific pathway, potentially leading to the identification of novel components or gene functions that manifest themselves as novel phenotypes.

Probes consisting of sequences first disclosed in SEQ ID NOS:1-23 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-23 can be utilized in microarrays or other assay formats, to screen collections of genetic material from patients who have a particular medical condition. These investigations can also be carried out using the sequences first disclosed in SEQ ID NOS:1-23 *in silico* and by comparing previously collected genetic databases and the disclosed sequences using computer software known to those in the art.

Thus, the sequences first disclosed in SEQ ID NOS:1-23 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligenucleotide sequence(s) first disclosed in the SEQ ID NOS: 1-23. Alternatively, a restriction map specifying the relative positions of restriction endonuclease digestion sites, or various palindrormic or other specific oligonucleotide sequences can be used to structurally describe a given sequence. Such restriction maps, which are typically generated by widely available computer programs (*e.g.*,the Univisity of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relative to one or more additional sequence (s) or cne or more restriction sites present in the disclosed sequence.

For oligonucleotide probes, highly stringent conditions may refer, e.g., to washing in 6xSSC/0.05% sodium pyrophosphate at 37°C (for 14-base oligos), 48°C (for 17-base oligos), 55°C (for 20-base oligos), and 60°C (or 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequances that are also useful for NHP gene regulation.

Inhibitory antisense or double stranded oligonucleotides can additionally comprise at least one modified base moiety which is selected from the group, including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carbomethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v)., wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group, including but not limited to, arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*: 6625-6641). The oligonucleotide is a 2'-0-methylribonucleotide (Inoue *et al.,* 1987, Nucl. Acids Res. *15*:6131-6148), or a chimeric RNA-DNA analogue (Inoue *et al.,* 1987, FEBS Lett. 215:327-330). Alternatively, double stranded RNA can be used to disrupt the expression and function of a targeted NHP.

Oligonucleotides of the invention can be synthesized by standard methods known in the art, *e.g.* by use of an automated DNA synthesizer (such as are commercially available from Biosearch, Applied Biosystems, etc.). As examples, phosphorothioate oligonucleotides can be synthesized by the method of Stein et al. (1988, Nucl. Acids Res. 16:3209), and methylphosphonate oligonucleotides can be prepared by use of controlled pore glass polymer supports (Sarin et al., 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook et al., 1989, Molecular Cloning, A Laboratory Manual (and periodic updates thereof), Cold Springs Harbor Press, NY; and Ausubel *et al.,* 1989, Current Protocols in Molecular Biology, Green Publishing Associates and Wiley Interscience, NY.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g*., splice acceptor and/or donor sites), etc., that can be used in diagnostics and pharmacogenomics.

Further, a NHP homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene. The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e*., one known, or suspected, to express a NHP gene, such as, for example, testis tissue). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g.*, Sambrook et al., 1989, supra.

A cDNA encoding a mutant NHP sequence can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal sequence. Using these two primers, the product is then amplified via PCR, optionally cloned into a suitable vector, and subjected to DNA sequent analysis through methods well known to those of skill in the art. By comparing the DNA sequence of the mutant NHP allele to that of a corresponding normal NHP allele, the mutation (s) responsible for the loss or alteration of function of the mutant NHP gene product can be ascertained.

Alternatively, a genomic library can be constructed using DNA obtained from an individual suspected of, or Known to carry, a mutant NHP allele (*e.g.,* a person manifesting a NHP- associated phenotype such as, for example, obesity, high blood pressure, arthritis, connective tissue disorders, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or, known to carry, such a mutant allele. In this manner, gene products made by the putatively mutant tissue can be expressed and screened using standard antibody screening techniques in conjunction with antibodies raised against normal NHP product, as described below (for screening techniques, see, for example, Harlow, E. and Lane, eds., 1988, "Antibodies: A Laboratory Manual", Cold Spring Harbor Press, cold- Spring Harbor, NY).

Additionally, screening can be accomplished by screening with labeled NHP fusion proteins, such as, for example, alkaline phosphatase-NHP or NHP-alkaline phosphatase fusion proteins. In cases where a NHP mutation results in an expression product with altered function (*e*.*g*., as a result of a missense or a frameshift mutation), polyclonal antibodies to NHP are likely to cross-react with a corresponding mutant NHP expression product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.*, antisense) ; (b) DNA expression vectors that contain any of the foregoing NHP coding sequences operatively associated with a regulatory element that directs the expression of the coding sequences (for example, baculo virus as described in U.S. Patent No. 5,869,336 ; (c) genetically engineered host cells that contain any of the foregoing NHB coding sequences operatively associated with a regulatory element that directs the expression of the coding sequences in the host cell; and (d) genetically engineered host cells that express an endogenous NHP sequence under the control of an exogenously introduced regulatory element (*i.e*., gene activation) or genetically engineered transcription factor. As used herein, regulatory elements include, but are not limited to, inducible and non-inducible promoters, enhancers, operators and other elements known to those skilled in the art that drive and regulate expression. Such regulatory elements include, but are not limited to,the cytomegalovirus (hCMV) immediate early gene, regulatable, viral elements (particularly retroviral LTR promoters), the early or late promoters of SV40 adenovirus, the lac system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of a NHP, as well as compounds or nucleotide constructs that inhibit expression of a NHP sequence (transcription factor inhibitors, antisense and ribozyme molecules, or open reading frame sequence or regulatory sequence replacement constructs), or promote the expression of a NHP (*e.g*., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHPs or NHP peptides, NHP fusion proteins, NHP nucleotide sequences, host cell expression systems, antibodies, antagonists, agonists and genetically engineered cells and non-human animals can be used for screening for drugs (or high throughput screening of combinatorial libraries) effective in the treatment of the symptomatic or phenotypic manifestations of perturbing the normal function of NHP in the body. The use of engineered host cells and/or animals may offer an advantage in that such systems allow not only for the identification of compounds that bind to the endogenous receptor for a NHP, but can also identify compounds that trigger NHP-mediated activities or pathways.

Finally, the NHP products can be used as therapeutics. For example, soluble derivatives such as NHP peptides/domains corresponding to NHP, NHP fusion protein products (especially NHP-Ig fusion proteins, *i.e*., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics a NHP could activate or effectively antagonize the endogenous NHP receptor. Nucleotide constructs encoding such NHP products can be used to genetically engineer host cells to express such products *in vivo*; these genetically engineered cells function as "bioreactors" in the body delivering a continuous supply of a NHP, a NHP peptide, or a NHP fusion protein to the body. Nucleotide constructs encoding functional NHP, mutant NHPs, as well as antisense and ribozyme molecules can also be used in "gene therapy" approaches for the modulation of NHP expression. Thus, the invention also encompasses pharmaceutical formulations and disclosed methods for treating biological disorders.

Various aspects of the invention are described in greater detail in the subsections below.

### 5.1 THE NHP SEQUENCES

The cDNA sequences and corresponding deduced amino acid sequences of the described NHPs are presented in the Sequence Listing. The NHP nucleotides were obtained from human cDNA libraries using probes and/or primers generated from human genomic sequence. Expression analysis has provided evidence that the described NHP can be expressed in a variety of human cells.

Several polymorphisms were identified including a A/G at the nucleotide position represented by, for example, position 58 of SEQ ID NO: 7 (which can result in a thr or ala at the region corresponding to amino acid (aa) position 20 of, for example, SEQ ID NO:8), a A/C at nucleotide position 1538 (which can result in a pro or gln at aa position 513), a C/T at position 1769 (which can result in an pro or leu at aa position 590), and a C/G at nucleotide position 1899 (which can result in a ser or arg at aa position 633).

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patents Nos. 5,830,721 and 5,837,458.

NHP gene products can also be expressed in non-human transgenic animals. Non-human Animals of any species, including, but mot limited to, worms, mice, rats, rabbits, guinea pigs, pigs, micro-pigs, birds, goats, and non-human primates, e.g., baboons, monkeys, and chimpanzees may be used to generate NHP transgenic animals.

Any technique known in the art may be used to introduce a NHP transgene into animals to produce the founder lines of transgenic animals. Such techniques include, but are not limited to, pronuclear microinjection (Hoppe, P.C. and Wagner, T.E., 1989, U.S. Pat. No. 4,873,191); retrovirus mediated gene transfer into germ lines (Van der Putten *et al.*, 1985, Proc. Natl. Acad. Sci., USA *82*:6148-6152); gene targeting in embryonic stem cells (Thompson *et al*., 1989, Cell *56*:313-321); electroporation of embryos (Lo, 1983, Mol Cell. Biol. *3*:1803-1814); and sperm-mediated gene transfer (Lavitrano *et al*., 1989, Cell *57*:717-723) ; etc. For a review of such techniques, see Gordon, 1989 Transgenic Animals, Intl. Rev. Cycol. 115:171-229.

The present invention provides for non-human transgenic animals that carry the NHP transgene in all their cells, as well as animals which carry the transgene in some, but not all their cells, *i.e.*, mosaics non-human animals or somatic cells non-human transgenic animals. The transgene may be integrated as a single transgene or in concatamers, *e.g*., head-to-head tandems or head-to-tail tandems. The transgene may also be selectively introduced into and activated in a particular cell type by following, for example, the teaching of Lasko *et al.*, 1992, Proc Natl. Acad. Sci. USA *89*:6232-6236. The regulatory sequences required for such a cell-type specific activation will depend upon the particular cell type of interest, and will be apparent to those of skill in the art.

When it is desired that a NHP transgene be integrated into the chromosomal site of the endogenous NHP gene, gene targeting is preferred. Briefly, when such a technique is to be utilized, vectors containing to nucleotide sequences homologous to the endogenous NHP gene are designed for the purpose of integrating, via homologous recombination with chromosomal sequences, into and disrupting the function of the nucleotide sequence of the endogenous NHP gene (*i.e*., non-human "knockout" animals).

The transgene can also be selectively introduced into a particular cell type, thus inactivating the endogenous NHP gene in only that cell type, by following, for example, the teaching of Gu *et al*., 1994, Science, *265*:103-106. The regulatory sequences required for such a cell-type specific inactivation will depend upon the particular cell type of interest, and will be apparent to those of skill in the art.

Once non-human transgenic animals have been generated, the expression of the recombinant NHP gene may be assayed utilizing standard techniques. Initial screening may be accomplished by Southern blot analysis or PCR techniques to analyze non-human animal tissues to assay whether integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and RT-PCR. Samples of NHP gene-expressing tissue may also be evaluated immunocytochemically using antibodies specific for the NHP transgene product.

### 5.2 NHPS AND NHP POLYPEPTIDES

The NHPs, NHP polypeptides, NHP peptide fragments, mutated, truncated, or deleted forms of NHP, and/or NHP fusion proteins can be prepared for a variety of uses. These uses include, but are not limited to, the generation of antibodies, as reagents in diagnostic assays, the identification of other cellular gene products related to a NHP, as reagents in assays for screening for compounds that can be used as pharmaceutical reagents useful in the therapeutic treatment of mental, biological, or medical disorders and disease. The described NHPs share similarity with a variety of proteases, including, but not limited to, proteases having thrombospondin repeats, disintegrins, aggrecanases, procollagen I N-proteinase, and metalloproteinases (especially zinc metalloproteases of the ADAMTS family).

The sequence Mating discloses the amino acid sequences encoded by the described NHP polynucleotides. The NHPs display initiator methionines in DNA sequence contexts consistent with translation initiation sites, and the ORFs display signal-like sequences which can indicate that the described NHP ORFs are secreted proteins or can be membrane associated.

The NHP amino acid sequences of the invention include the amino acid sequences presented in the Sequence Listing as well as analogues and derivatives thereof. Further, corresponding NHP homologues from other species are encompassed by the invention. In fact, any NHPs encoded by a NHP nucleotide sequence described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the sequence Listing, is generically representative of the well known nucleic acid "triplet" codon, or in many cases codons that can encode the amino acid. As such, as contemplated herein, the amino acid sequences presented in the Sequence Listing, when taken together with the genetic code (see, for example, Table 4-1 at page 109 of "Molecular Cell Biology" 1986, J. Darnell at al eds., Scientific American Books, New York, NY are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e.g*., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent expression product. Amino acid substitutions can be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, a NHP peptide or NHP polypeptide is thought to be a soluble or secreted molecule, the peptide or polypeptide can be recovered from the culture media. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of a NHP, but to assess biological activity, *e.g.*, in drug screening assays.

The expression systems that may be used for purposes of the invention include, but are not limited to, microorganisms such as bacteria (*e.g., E. coli, B. subtilis*) transformed with recombinant bacteriophage DNA, plasmid DNA or cosmid DNA expression vectors containing NHP, nucleotide sequences; yeast (*e.g., Saccharomyces, Pichia*) transformed with recombinant yeast expression vectors containing NHP encoding, nucleotide sequences; insect cell systems infected with recombinant virus expression vectors (*e.g*., baculovirus) containing NHP sequences; plant cell systems injected with recombinant virus expression vectors (*e.g*., cauliflower mosaic virus, CaMV; tobacco mosaic virus, TMV) or transformed with recombinant plasmid expression vectors (*e.g.*, Ti plasmid) containing NHP nucleotide sequences; or mammalian cell systems (*e.g.*, COS, CHO, BHK, 293, 3T3) harboring recombinant expression constructs containing promoters derived "from" the genome of mammalian cells (*e.g.,* metallothibheih promoter) or from mammalian viruses (*e.g.*, the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of and/or containing a NHP, or for raising antibodies to a NHP, vectors that direct the expression of high levels of fusion protein products that are readily purified may be desirable. Such vectors include, but are not limited, to the *E. coli* expression vector pUR278 (Ruther *et al.,* 1983, EMBO J. *2*:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109 Van Heeke & schuster, 1989, J. Biol. Chem. *264*:5503-5509); and the like. pGEX vectors (Pharmacia or American Type Culture Collection) can also be used to express foreign polypeptides as fusion proteins with glutathione S-taransferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target expression product can be released from the GST moiety.

In an insect system, *Autographa californica* nuclear polyhedrosis virus (AcNPV) is used as a vector to express foreign polynucleotide seqeunces. The virus grows in *Spodoptera frugiperda* cells. A NHP coding sequence can be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e.,* virus lacking the proteinaceous coat coded for by the polyhedrin gene). These recombinant viruses are then used to infect Spodoptera frugiperda cells in which the inserted sequence is expressed (*e.g.,* see Smith *et al.,* 1983, J. Virol. 46: 584; U.S. Patent No . 4,215,051).

In mamalian host cells, a number of viral-based expression systems may be utilized. In cases where an adenovirus is used as an expression vector, the NPH nucleotide sequence of interest may be ligated to an adenovirus. transcription/translation control complex, *e*.*g*., the late promoter and tripartite leader sequence. This chimeric sequence may then be inserted in the adenovirus genome by in vitro or *in vivo* recombination. Insertion in a non-essential region of the viral genome (*e*.*g*., region E1 or E3) will result in a recombinant virus that is viable and capable of expressing a NHP product in infected hosts (*e.g*., See Logan & Shenk, 1984, Proc. Nat1. Acad. Sci. USA *81*:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter *et a*l., 1987, Methods in Enzymol. *153*:516-544).

In addition, a host cell strain may be chosen that modulates the expression of the inserted sequences, or modifies and processes the expression product in the specific fashion desired. Such modifications (*e.g.*, glycosylation) and processing (*e.g*., cleavage) of protein products may be important for the function of the protein. Different host cells have characteristic and specific mechanisms for the post-translational processing and modification of proteins and expression products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the expression product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e*.*g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express a NHP product. Such engineered cell lines may be particularly useful in screening and evaluation of compounds that affect the endogenous activity of a NHP product.

A number of selection systems may be used, including but not limited to the herpes simplex virus thymidine kinase (Wigler, *et a*l., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et a*l., 1980, Cell *22*:817) genes, which can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA *77*:3567; O'Hare, *et al*., 1981, Proc. Natl. Acad. Sci. USA *78*:1527); gpt, which confers resistance to mycophenolic acid (Mulligan & Berg, 1981, Proc. Natl. Acad. Sci. USA *78*:2072); neo, which confers resistance to the aminoglycoside G-418 (Colberre-Garapin, *et al.,* 1981, J. Mol. Biol. 150:1); and hygro, which confers resistance to hygromycin (Santerre, *et al.,* 1984, Gene *30*:147).

Alternatively, any fusion protein can be readily purified by utilizing an antibody specific for the fusion protein being expressed. For example, a system described by Janknecht et al. allows for the ready purification of non-denatured fusion proteins expressed in human cell lines (Janknecht, *et al*., 1991, Proc. Natl. Acad. Sci. USA *88*:8972-8976). In this system, the sequence of interest is subcloned into a vaccinia recombination plasmid such that the sequence's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively, targeting of NHP or its nucleic acid sequence might be achieved using liposome or lipid complex based delivery systems. Such technologies are described in "Liposomes: A Practical Approach", New, R.R.C. , ed., Oxford University Press, New York and in U.S. Patents Nos. 4,594,595, 5,459,127, 5,948 767 and 6,110,490 and their respective disclosures.

Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain to facilitate passage across cellular membranes and can optionally be engineered to include nuclear localization.

### 5.3 ANTIBODIES TO NHP PRODUCTS

Antibodies that specifically recognize one or more epitopes of a NHP, or epitopes of conserved variants of a NHP, or peptide fragments, of a NHP are also disclosed.

Such antibodies include, but are not limited to, polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of a NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP expression product. Additionally, such antibodies can be used in conjunction with gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods

For the production of antibodies, various host animals may be immunized by injection with a NHP, an NHP peptide (*e.g.,* one corresponding to a functional domain of a NHP), truncated NHP polypeptides (NHP in which one or more domains have been deleted), functional equivalents of a NHP, or mutated variants of a NHP. Such host animals may include, but are not limited to, pigs, rabbits, mice, goats, and rats, to name but a few. Various adjuvants may be used to increase the immunological response, depending on the host species, including but not limited to, Freund's adjuvant (complete and incomplete), mineral salts such as aluminum hydroxide or aluminum phosphate, chitosan, surface active substances such as lysolecithin, pluronic polyols, polyanions, peptides, oil emulsions, and potentially useful human adjuvants such as BCG (bacille Calmette-Guerin) and *Corynebacterium parvum*. Alternatively, the immune response could be enhanced by combination and or coupling with molecules such as keyhole limpet hemocyanin, tetanus toxoid, diphtheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature *256* :495-497; and U.S. Patent No. 4,375,110), the human B-cell hybridoma technique (Kosbor *et al.*, 1983, Immunology Today *4*:72; Cole *et al*., 1983, Proc. Natl. Acad. Sci. USA *80*:2026-2030), and the EBV-hybridoma technique (Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition techniques developed for the production of "chimeric antibodies" (Morrison *et al.,* 1984, Proc. Natl. Acad. Sci., *81*: 6851-6855; Neuberger *et al*., 1984, Nature, *312*:604-608; Takeda *et al.,* 1985, Nature, *314*:452-454) by spacing the genes from a mouse antibody molecule of appropriate antigen specificity together with genes from a human antibody molecule of appropriate biological activity can be used (see U.S. Patents Nos. 5,877,397 and 6,075,181).

A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures.

Also encompassed by the present invention is the use of fully humanized monoclonal antibodies as described in US Patent No. 6,150,584 and respective disclosures.

Alternatively, techniques described for the production of single chain antibodies. (U.S. Patent 4,946,778; Bird, 1988, Science *242*:423-426; Huston *et al*., 1988, Proc. Natl. Acad. Sci. USA 85:5879-5883; and Ward *et al*., 1989, Nature *341*: 544-546) can be adapted to produce single chain antibodies against NHP expression products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragment. Alternatively, Fab expression libraries may be constructed (Huse *et al.,* 1989, Science, *246*:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, *e.g*., Greenspan & Bona, 1993, FASEB J *7(5)*:437-444; and Nissinoff, 1991, J. Immunol. *147(8)* :2429-2438). For example, antibodies which bind to a NHP domain and competitively inhibit the binding of NHP to its cognate receptor can be used to generate anti-idiotypes that "mimic" a NHP and, therefore, bind and activate or neutralize a receptor. Such anti-idiotypic antibodies or Fab fragments of such, anti-idiotypen can be used in therapeutic regimens involving a NHP signaling pathway

Additionally, given the high degree of relatedness of mammalian NHPs, the presently described knock-out mice (having never seen NHP, and thus never been tolerized to NHP) have a unique utility, as they can be advantageously applied to) the generation of antibodies against the disclosed mammalian HNP (i.e., NHP will be inmunogenic in NHP knock-out animals).

## Claims

1. An isolated nucleic acid molecule comprising a nucleotide sequence encoding an amino acid sequence drawn from the group consisting of SEQ ID NOS: 2, 4, 6, 8, 10, 12, 14, 16, 18, 20 and 22.

2. An isolated nucleic acid molecule comprising a nucleotide sequence that:
(a) encodes the amino acid sequence shown in SEQ ID NO: 20; and
(b) hybridizes under stringent conditions to the nucleotide sequence of SEQ ID NO: 19 or the complement thereof.

3. An isolated nucleic acid molecule encoding the amino acid sequence presented in SEQ ID NO: 20.

4. An isolated nucleic acid molecule encoding the amino acid sequence presented in SEQ ID NO: 22.

5. An isolated nucleic acid molecule according to any one of claims 1 to 4 wherein said nucleotide sequence is present in cDNA.

6. A DNA vector comprising a nucleotide sequence according to any one of claims 1 to 4.

7. A polypeptide encoded by a nucleic acid as claimed in any one of claims 1 to 5.

8. An F(ab')2 antibody derivative that specifically recognises a polypeptide as claimed in claim 7.

9. An isolated host cell that comprises a nucleic acid as claimed in any one of claims 1 to 5, or a DNA vector as claimed in claim 6 and is capable of expressing the polypeptide encoded by said nucleic acid, optionally on the surface of said host cell.

10. A non-human transgenic animal that is capable of expressing a nucleic acid as claimed in any one of claims 1 to 5, or that is any optionally conditional "knock-out" non-human animal that does not express a functional polypeptide as claimed in claim 7.

11. Use of a nucleic acid as claimed in any one of claims 1 to 5, a polypeptide as claimed in claim 7, an antibody as claimed in claim 8, a host cell as claimed in claim 9, or a transgenic animal as claimed in claim 10, in a method for screening compounds for the ability to modulate the expression of a nucleic acid as claimed in any one of claims 1 to 5, or a polypeptide as claimed in claim 7, or that modulate activity of a polypeptide as claimed in claim 7, for example, for high throughput screening of combinatorial libraries.

## Patentansprüche

1. Ein isoliertes Nukleinsäuremolekül aufweisend eine Nukleotidsequenz, die für eine Aminosäuresequenz codiert, welche aus der Gruppe gezogen wird bestehend aus den SEQ ID NOs: 2, 4, 6, 8, 10, 12, 14, 16, 18, 20 und 22.

2. Isoliertes Nukleinsäuremolekül, das eine Nukleotidsequenz umfasst, die:
(a) für die Aminosäuresequenz, die in SEQ ID NO: 20 gezeigt ist, codiert; und
(b) unter stringenten Bedingungen mit der Nukleotidsequenz von SEQ ID NO: 19 oder dem Komplement davon hybridisiert.

3. Isoliertes Nukleinsäuremolekül, das für die in SEQ ID NO: 20 präsentierte Aminosäuresequenz codiert.

4. Isoliertes Nukleinsäuremolekül, das für die in SEQ ID No: 22 präsentierte Aminosäuresequenz codiert.

5. Isoliertes Nukleinsäuremolekül nach einem der Ansprüche 1 bis 4, wobei die Nukleotidsequenz in cDNA vorhanden ist.

6. DNA-Vektor, der eine Nukleotidsequenz nach einem der Ansprüche 1 bis 4 umfasst.

7. Polypeptid, das durch eine Nukleinsäure nach einem der Ansprüche 1 bis 5 codiert wird.

8. Ein F(ab')2 Antikörperderivat, das spezifisch ein Polypeptid nach Anspruch 7 erkennt.

9. Isolierte Wirtszelle, die eine Nukleinsäure nach einem der Ansprüche 1 bis 5 oder einen DNA-Vektor nach Anspruch 6 umfasst, und zur Expression des Polypeptids, das durch die Nukleinsäure codiert wird, optional an der Oberfläche der Wirtszelle, fähig ist.

10. Nicht-menschliches genetisch verändertes Tier, das eine Nukleinsäure nach Anspruch 1 oder 2 exprimiert, oder das ein optional konditionelles" Knock-Out-Tier" ist, das kein funktionelles Polypeptid nach Anspruch 7 exprimiert.

11. Verwendung einer Nukleinsäure nach einem der Ansprüche 1 bis 5, eines Polypeptids nach Anspruch 7, eines Antikörpers nach Anspruch 8, einer Wirtszelle nach Anspruch 9 oder eines transgenen Tieres nach Anspruch 10, in einem Verfahren zum Screening von Verbindungen nach der Fähigkeit die Expression einer Nukleinsäure nach einem der Ansprüche 1 bis 5, oder eines Polypeptids nach Anspruch 7 zu modulieren, oder die die Aktivität eines Polypeptids nach Anspruch 7 modulieren, zum Beispiel im Hochdurchsatzscreening von kombinatorischen Bibliotheken.

## Revendications

1. Une molécule d'acide nucléique isolée comprenant une séquence nucléotidique qui code pour la séquence d'acide aminé appartenant au groupe consistant en SEQ ID N° 2, 4, 6, 8, 10, 12, 14, 16, 18, 20 et 22.

2. Une molécule d'acide nucléique isolée comprenant une séquence nucléotidique qui:
(a) code pour la séquence d'acide aminé représentée par SEQ ID N° 20; et
(b) s'hybride dans des conditions stringentes à la séquence nucléotidique de SEQ ID N° 19 ou de son complément.

3. Une molécule d'acide nucléique isolée qui code pour la séquence d'acide aminé représentée par SEQ ID N° 20.

4. Une molécule d'acide nucléique isolée qui code pour la séquence d'acide aminé représentée par SEQ ID N° 22.

5. Une molécule d'acide nucléique isolée telle que revendiquée dans une quelconque des revendications 1 à 4, dans laquelle la dite séquence nucléotidique est présente dans l'ADNc.

6. Une molécule d'acide nucléique isolée comprenant une séquence nucléotidique telle que revendiquée dans une quelconque des revendications 1 à 4.

7. Un polypeptide codé par un acide nucléique tel que revendiqué dans une quelconque des revendications 1 à 5.

8. Un dérivé F(ab')2 d'anticorps qui reconnaît spécifiquement un polypeptide tel que revendiqué dans la revendication 7.

9. Une cellule hôte isolée qui comprend un acide nucléique selon l'une quelconque des revendications 1 à 5, ou un vecteur ADN selon la revendication 6 et qui est capable d'exprimer le polypeptide codé par ledit acide nucléique, éventuellement à la surface de la cellule.

10. Un animal non-humain modifié génétiquement qui est capable d'exprimer un acide nucléique tel que revendiqué dans une quelconque des revendication 1 à 5 ou qui est éventuellement un animal non humain "knock-out" conditionnel qui n'exprime pas un polypeptide fonctionnel tel que revendiqué dans la revendication 7.

11. Utilisation d'un acide nucléique selon une quelconque des revendications 1 à 5, d'un polypeptide selon la revendication 7, d'un anticorps selon la revendication 8, d'une cellule hôte selon la revendication 9, ou d'un animal non humain modifié génétiquement selon la revendication 10, dans une méthode de criblage de composés pour leur capacité à moduler l'expression d'un acide nucléique selon une quelconque des revendications 1 à 5 ou d'un polypeptide selon la revendication 7, ou qui module l'activité d'un polypeptide selon la revendication 7, par exemple pour un criblage à haut rendement de bibliothèques combinatoires.
